# EUROPEAN PATENT APPLICATION

(11) **EP 2 286 804 A1**
(43) Date of publication of application: **23.02.2011**
(21) Application number: 10183305.1
(22) Date of filing: 13.05.2004
(51) Int. Cl.: A61K 31/00, A61K 31/4439, A61K 38/00, A61K 38/48, A61K 9/00, A61P 19/00

(54) **A method of treating degenerative disc disease**

(30) Priority: 13.08.2003 US 640412; 31.07.2003 US 631487; 30.06.2003 US 610355; 06.06.2003 US 456948; 13.05.2003 US 470098 P
(62) Divisional of application: 04776014.5
(71) Applicant: DePuy Spine, Inc., Raynham, MA 02767-0350 (US)
(72) Inventor: Serhan, Hassan, South Easton, MA 02375 (US); Dimauro, Thomas M, Southborough, MA 01772 (US); Attawia, Mohamed, Canton, MA 02021 (US); Grace, Melissa, Raynham, MA 02767 (US); Kadiyala, Sudhakar, Newton, MA 02459 (US); Urbahns, David, Barrington, RI 02806 (US); Bruder, Scott, Sudbury, MA 01776 (US); Collins, Gregory, Esat Sandwich, MA 02537 (US); Brown, Laura J, Hamilton Square, MA 08690 (US); Geesin, Jeff, Doylestown, PA 18901 (US); Plouhar, Pamela L, South Bend, IN 46614 (US); Smith, Catherine, East Falmouth, MA 02536 (US); Siekierka, John, Towaco, NJ 07082 (US)
(74) Representative: Belcher, Simon James

(57) **Abstract**

Described herein are methods of treating degenerative disc disease by injecting an antagonist such as a high specificity cytokine antagonist, a high affinity antagonist of MMP's, a high specific p38 kinase inhibitor, and a cycline compound into a diseased intervertebral disc.

## Description

### RELATED APPLICATIONS

This application is a continuation-in-part of U.S. Application No. 10/640,412, filed August 13, 2003 and U.S. Application No. 10/631,487, filed July 31, 2003, which is a continuation-in-part of U.S. Application No. 10/610,355, filed June 30, 2003, which is a continuation in part of U.S. Application No. 10/456,948, filed June 6, 2003, which claims the benefit of U.S. Provisional Application No. 60/470,098, filed May 13, 2003. The entire teachings of the above applications are entirely incorporated herein by reference.

### BACKGROUND OF THE INVENTION

The natural intervertebral disc contains a jelly-like nucleus pulposus surrounded by a fibrous annulus fibrosus. Under an axial load, the nucleus pulposus compresses and radially transfers that load to the annulus fibrosus. The laminated nature of the annulus fibrosus provides it with a high tensile strength and so allows it to expand radially in response to this transferred load.

In a healthy intervertebral disc, cells within the nucleus pulposus produce an extracellular matrix (ECM) containing a high percentage of proteoglycans. These proteoglycans contain sulfated functional groups that retain water, thereby providing the nucleus pulposus with its cushioning qualities. These nucleus pulposus cells may also secrete small amounts of cytokines as well as matrix metalloproteinases (MMPs). These cytokines and MMPs help regulate the metabolism of the nucleus pulposus cells.

In some instances of degenerative disc disease (DDD), gradual degeneration of the intervertebral disc is caused by mechanical instabilities in other portions of the spine. In these instances, increased loads and pressures on the nucleus pulposus cause the cells within the disc (or invading macrophages) to emit larger than normal amounts of the above-mentioned cytokines. In other instances of DDD, genetic factors or apoptosis can also cause the cells within the nucleus pulposus to emit toxic amounts of these cytokines and MMPs. In some instances, the pumping action of the disc may malfunction (due to, for example, a decrease in the proteoglycan concentration within the nucleus pulposus), thereby retarding the flow of nutrients into the disc as well as the flow of waste products out of the disc. This reduced capacity to eliminate waste may result in the accumulation of high levels of proinflammatory cytokines and/or MMPs that may cause nerve irritation and pain.

As DDD progresses, toxic levels of the cytokines and MMPs present in the nucleus pulposus begin to degrade the extracellular matrix. In particular, the MMPs (as mediated by the cytokines) begin cleaving the water-retaining portions of the proteoglycans, thereby reducing their water-retaining capabilities. This degradation leads to a less flexible nucleus pulposus, and so changes the loading pattern within the disc, thereby possibly causing delamination of the annulus fibrosus. These changes cause more mechanical instability, thereby causing the cells to emit even more cytokines, typically thereby upregulating MMPs. As this destructive cascade continues and DDD further progresses, the disc begins to bulge ("a herniated disc"), and then ultimately ruptures, causing the nucleus pulposus to contact the spinal cord and produce pain.

### SUMMARY OF THE INVENTION

The present inventors have developed a number of procedures for efficaciously treating degenerative disc disease by drug therapy.

In accordance with one embodiment of the present invention, the present inventors have developed a method of treating an intervertebral disc in which an antagonist (e.g., a protein) is administered transdiscally (i.e., the target tissue is a degenerating disc). As used herein, an antagonist refers to an inhibitor for example, an inhibitor of a protein or proteins (such as cytokines and MMPs), such as proteins emitted from nucleus pulposus and/or annulus fibrosus cells. Such proteins can be emitted in toxic amounts as a result of DDD. In some embodiments, the antagonist is selected from the group consisting a high specificity cytokine antagonist (HSCA), a high affinity anti-matrix metalloproteinase (HAAMMP), an antagonist of p38 kinase or a cycline compound.

There are believed to be several advantages to directly transdiscally administering such antagonists to a targeted disc:
First, HSCAs inhibit the activity of cytokines. HAAMMPs and cycline compounds inhibit the activity of MMPs. Since it is known that cytokines (such as interleukins and TNF-α) and MMPs play primary roles in the degradation of the extracellular matrix (ECM) of the nucleus pulposus, injecting an antagonist directly into the disc in a therapeutically effective amount can prevent the cytokines and/or MMPs from causing any further ECM degradation. In effect, the transdiscal adminstration of the antagonists (e.g., HAAMMPs) helps arrest the aging process of the degenerating disc. Accordingly, the present invention seeks to treat the degenerative disc at a much earlier stage of DDD and thereby prevents degradation of the ECM.
Second, cycline compounds and some HSCAs inhibit the activity of TNF-α. It is further known that nerve ending nociceptors are present within the annulus fibrosus, and that TNF-α participates in the irritation of nerves. It is believed that injecting a TNF-α antagonist (such as a cycline compound) into the disc space also prevents the TNF-α from causing nerve irritation within the disc. Thus, the pain attributed to irritation of these nerves can be efficiently eliminated.
Third, since some antagonists are specific, they do not inhibit non-targeted cells, tissue or proteins. In addition, the antagonist may be combined with other (i.e., additional) therapeutic agents (such as growth factors) that can also be injected into the disc without reducing the effectiveness of those agents.
Fourth, since the annulus fibrosus portion of the disc comprises a dense fibrosus structure, this outer component of the disc may provide a suitable depot for the antagonist, thereby increasing its half-life in the disc.
Fifth, since at least MMPs may be secreted by at least one of invading macrophages, nucleus pulposus cells or annulus fibrosus cells, transdiscal injection of cycline compounds will advantageously attack the MMPs at their source of origination.

Accordingly, in one aspect of the present invention, there is provided a method of treating degenerative disc disease in an intervertebral disc having a nucleus pulposus, comprising transdiscally administering a formulation comprising an effective amount of a formulation, wherein the antagonist is selected from the group consisting of: a) a high specificity cytokine antagonist, b) a high affinity anti matrix metalloproteinase (HAAMMP), c) an antagonist of p38 kinase (i.e., a p38 kinase inhibitor), and d) a cycline compound.

In one embodiment, there is provided a method of treating degenerative disc disease in an intervertebral disc having a nucleus pulposus, comprising transdiscally administering an effective amount of a formulation which comprises: a) a high specificity cytokine antagonist, b) a high affinity anti-matrix metalloproteinase (HAAMMP), c) an antagonist of p38 kinase, and d) a cycline compound.

In one embodiment, the antagonist, e.g., a p38 kinase inhibitor, is administered in a dosage to produce a local tissue concentration of between about 5 µg/kg and about 50 µg/kg. In one embodiment, the formulation is administered in an amount of less than about 1 cc. In one embodiment, the antagonist is present in the formulation in an amount of at least about 100 mg/ml. In one embodiment, the antagonist is present in the formulation in an amount of no more than about 0.5 mg.

In some embodiments of the invention described herein, the formulation further comprises a sustained release device, e.g., a sustained release device comprising a hydrogel. In one embodiment, the sustained release device provides controlled release. In another embodiment, the sustained release device provides continuous release. In yet another embodiment, the sustained release device provides intermittent release.

In one embodiment, the sustained release device comprises a biosensor. In one embodiment, the sustained release device comprises a plurality of microspheres. In one embodiment, the sustained release device comprises an inflammatory-responsive delivery system.

In another embodiment, the antagonist is predominantly released from the sustained delivery device by its diffusion through the sustained delivery device. In yet another embodiment, the sustained delivery device is a polymer. In one embodiment, the antagonist is predominantly released from the sustained delivery device by biodegradation of the sustained delivery device.

In one embodiment, the formulation further comprises at least one additional (e.g., a second, third or fourth) therapeutic agent. In one embodiment, at least one additional therapeutic agent is co-administered (for example, sequentially or simultaneously) with the antagonist. In one embodiment, the formulation is administered in an amount effective to reduce pain. In one embodiment, the formulation is administered in an amount effective to inhibit degradation of an extracellular matrix of the nucleus pulposus. The additional therapeutic agent can comprise any additional agent disclosed herein, including any antagonist disclosed herein.

In one embodiment, the additional therapeutic agent is a growth factor. The growth factor can be present in an amount effective to repair disc tissue. In one embodiment, the growth factor is a TGF-β. In one embodiment, the growth factor is provided by platelet concentrate.

In one embodiment, the additional therapeutic agent is plasmid DNA.

In one embodiment, the nucleus pulposus is removed prior to transdiscally administering the formulation.

In one embodiment, the formulation is administered through a drug pump. In yet another embodiment, the formulation is administered through a needle. In one embodiment, the needle has a maximum gauge of 24 gauge. In yet another embodiment, the formulation is administered in a volume of between about 0.03 ml and about 0.3 ml.

In some embodiments of the invention, the administration comprises providing the formulation in a patch attached to an outer wall of the annulus fibrosus. In another embodiment, the administration comprises providing the formulation in a depot at a location closely adjacent to an outer wall of the annulus fibrosus. In yet another embodiment, the administration comprises providing the formulation in a depot at a location closely adjacent to an endplate of an adjacent vertebral body. In another embodiment, the formulation is provided closely adjacent the outer wall of the annulus fibrosus. In yet another embodiment, the formulation is injected into the nucleus pulposus. In one embodiment, the formulation is injected into the annulus fibrosus.

In one embodiment, the degenerating disc is an intact disc. In another embodiment, the degenerating disc is a ruptured disc. In one embodiment, the degenerating disc is delaminated. In one embodiment, the degenerating disc has fissures.

There is also provided a formulation for treating degenerative disc disease, comprising an antagonist selected from the group consisting of:
a) a high specificity cytokine antagonist,
b) a high affinity anti-matrix metalloproteinase HAAMMP,
c) an antagonist of p38 kinase,
d) a cycline compound; and
d) an additional therapeutic agent selected from the group consisting of:
   i) a growth factor, and
   ii) plasmid DNA.

There is also provided a method of therapeutically treating a degenerating intervertebral disc, comprising the steps of:
a) determining a level of a pro-inflammatory protein within the disc,
b) comparing the level against a pre-determined level of the pro-inflammatory protein, and
c) injecting an antagonist into the disc, wherein the antagonist is selected from the group consisting of:
   i) a high specificity cytokine antagonist,
   ii) a high affinity anti-matrix metalloproteinase HAAMMP,
   iii) a high specificity antagonist of p38 kinase inhibitor,
      and
   iv) a cycline compound.

In one embodiment, the proinflammatory protein is an interleukin. In one embodiment, the predetermined level for the interleukin is at least 100 µg/ml.

In onebodiment, the proinflammatory protein is an interleukin-6. In one embodiment, the predetermined level for the interleukin-6 is at least 100 pg/ml. In one embodiment, the predetermined level for the interleukin-6 is at least 250 pg/ml.

In one embodiment, the proinflammatory protein is an interleukin-8. In one embodiment, the predetermined level for the interleukin-8 is at least 500 pg/ml.

In one embodiment, the proinflammatory protein is PGE2. In one embodiment, the predetermined level for PGE2 is at least 1000 pg/ml.

In one embodiment, the proinflammatory protein is TNF-α. In one embodiment, the predetermined level for TNF-α is at least 20 pg/ml. In one embodiment, the predetermined level for TNF-α is at least 30 pg/ml. In one embodiment, the predetermined level for TNF-α is at least 1000 pg/disc.

There is also provided a method of preventing degeneration of an intervertebral disc in a human individual, comprising:
a) determining a genetic profile of the individual;
b) comparing the profile of the individual against a pre-determined genetic profile level of at-risk humans;
c) determining that the individual is an at-risk patient; and
d) injecting an antagonist into a disc of the individual, wherein the antagonist is selected from the group consisting of:
   i) a high specificity cytokine antagonist,
   ii) a high affinity antagonist of MMPs,
   iii) a high specificity antagonist of p38 kinase, and
   iv) a cycline compound.

There is also provided a method of treating degenerative disc disease in an intervertebral disc having a nucleus pulposus, comprising transdiscally administering an effective amount of a formulation comprising an antagonist of COX-2 enzyme, high specificity antagonist of NO synthase, high specificity antioxidant, high specificity anti-proliferative agent and/or high specificity anti-apoptotic agent into an intervertebral disc.

In some embodiments, formulation comprises a cytokine compound and at least one highly specific antagonist (HSA) selected from the group consisting of:
i) an inhibitor of a pro-inflammatory interleukin;
ii) an inhibitor of TNF-α synthesis;
iii) an inhibitor of membrane-bound TNF-α;
iv) an inhibitor of a natural receptor of TNF-α;
v) an inhibitor of NO synthase;
vi) an inhibitor of PLA2 enzyme;
vii) an anti-proliferative agent;
viii) an anti-oxidant;
ix) an apoptosis inhibitor selected from the group consisting of EPO mimetic peptides, EPO mimetibodies, IGF-I, IGF-II, and caspase inhibitors;
x) an inhibitor of MMPs; and xi) a p38 kinase inhibitor.

### STATEMENTS OF INVENTION

According to the present invention there is provided a method of treating degenerative disc disease in an intervertebral disc having a nucleus pulposus, comprising transdiscally administering an effective amount of a formulation into an intervertebral disc, wherein the formulation-comprises an antagonist selected from the group consisting of : a) a high specificity cytokine antagonist, b) a high affinity anti-matrix metalloproteinase (HAAMMP), c) a p38 kinase inhibitor, and d) a cycline compound.

The method maybe provided wherein the p38 kinase inhibitor is selected from the group consisting of: i) diaryl imidizole ; ii) N, N'-diary urea; iii) N, N-diary urea; iv) benzophenone; v) pyrazol ketone; vi) indole amide; vii) diamides; viii) quinazoline ; ix) pyrimido [4,5-d] pyrimidinone; and x) pyridylamino-quinazolines.

The method maybe provided wherein the p38 kinase inhibitor is a 1-aryl-2- pyridinyl heterocycle.

The method maybe provided wherein the p38 kinase inhibitor has at least 3 cyclic groups.

The method maybe provided wherein the antagonist is water insoluble or substantially water insoluble.

The method maybe provided wherein the HAAMMP is a TIMP.

The method maybe provided wherein the HAAMMP is an anti-aggrecanase.

The method maybe provided wherein the HAAMMP is a specific antagonist of an MMP selected form the group consisting of collagenase MMP; stromelysin MMP; and gelatinase MMP.

The method maybe provided wherein the HAAMMP is a specific antagonist of an MMP selected from the group consisting of MMP-2, MMP-3 and MMP-8.

The method maybe provided wherein the cycline compound is selected from the group consisting of doxycycline, lymecycline, oxicycline compound, tetracycline, minocycline, chemically modified tetracycline and KB-R7785.

The method maybe provided wherein the formulation is predominantly released from the sustained delivery device by its diffusion through the sustained delivery device or by biodegradation of the sustained delivery device.

The method maybe provided wherein the formulation further comprises at least one additional therapeutic agent.

The method maybe provided wherein the formulation is administered in an amount effective to reduce pain.

The method maybe provided wherein the formulation is administered in an amount effective to inhibit degradation of an extracellular matrix of the nucleus pulposus.

The method maybe provided wherein the antagonist is administered in a dosage to produce a local tissue concentration of between about 5 Rg/kg and about 50 llg/kg.

The method maybe provided wherein the formulation is administered in an amount of less than about 1 cc.

The method maybe provided wherein the antagonist is present in the formulation in an amount of at least about 100 mg/ml.

The method maybe provided wherein the formulation further comprises a sustained release device or glycosaminoglycans.

The method maybe provided wherein the formulation is provided closely adjacent the outer wall of the annulus fibrosus.

The method maybe provided wherein the antagonist is present in the formulation in an amount of no more than about 0.5 mg.

The method maybe provided wherein the formulation further comprises a growth factor present in an amount effective to repair disc tissue.

The method maybe provided wherein the formulation is administered through a drug pump.

The method maybe provided wherein the formulation is injected into the nucleus pulposus.

The method maybe provided wherein the formulation is injected into the annulus fibrosus.

The method maybe provided wherein a portion of the nucleus pulposus is removed prior to transdiscally administering the formulation.

The method maybe provided wherein the formulation is administered through a needle.

The method maybe provided wherein the formulation is administered in a volume of between about 0.03 ml and about 0. 3 ml.

The method maybe provided wherein the administration comprises: a) providing the formulation in a patch attached to an outer wall of the annulus fibrosus; b) providing the formulation in a depot at a location closely adjacent to an outer wall of the annulus fibrosus; and c) providing the formulation in a depot at a location closely adjacent to an endplate of an adjacent vertebral body.

The method maybe provided wherein wherein the degenerating disc is an intact disc.

The method maybe provided wherein the degenerating disc is a ruptured disc.

The method maybe provided wherein the degenerating disc is delaminated.

The method maybe provided wherein the degenerating disc has fissures.

According to another aspect of the invention there is provided a formulation for treating degenerative disc disease, comprising an antagonist, wherein the antagonist is selected from the group consisting of : a) a high specificity cytokine antagonist, b) a high affinity anti-matrix metalloproteinase HAAMMP, c) a p38 kinase inhibitor, d) a cycline compound; and d) an additional therapeutic agent selected from the group consisting of : i) a growth factor, ii) plasmid DNA, iii) a high specificity antagonist of a pro-inflammatory interleukin, iv) a high specificity antagonist of TNF-a, v) a high specificity antagonist of PLA2, vi) is a high specificity anti-proliferative agent, and vii) a highly specific apoptosis inhibitor.

According to another aspect of the invention there is provided a method of therapeutically treating a degenerating intervertebral disc, comprising the steps of : a) determining a level of a pro-inflammatory protein within the disc, b) comparing the level against a pre-determined level of the pro- inflammatory protein, and c) injecting an antagonist into the disc, wherein the formulation is selected from the group consisting of : i) a high specificity cytokine antagonist, ii) a high affinity anti-matrix metalloproteinase HAAMMP, iii) a p38 kinase inhibitor, and iv) a cycline compound.

The method maybe provided wherein the proinflammatory protein is an interleukin-6 or interleukin-8.

The method maybe provided wherein the predetermined level for TNF-a is at least 20 pg/ml, at least 30 pg/ml, or at least 1000 pg/disc.
A method of preventing degeneration of an intervertebral disc in a human individual, comprising : a) determining a genetic profile of the individual ; b) comparing the profile of the individual against a pre-determined genetic profile level of at-risk humans; c) determining that the individual is an at-risk patient; and d) injecting an antagonist into a disc of the individual, wherein the antagonist is selected from the group consisting of : i) a high specificity cytokine antagonist, ii) a high affinity antagonist of MMPs, iii) a p38 kinase inhibitor, and iv) a cycline compound.

A method of treating degenerative disc disease in an intervertebral disc having a nucleus pulposus, comprising transdiscally administering an effective amount of a formulation comprising an antagonist of COX-2 enzyme, an antagonist of NO synthase, a high specificity antioxidant, a high specificity anti-proliferative agent, or a high specificity anti-apoptotic agent into an intervertebral disc.

The method maybe provided wherein the high specificity cytokine antagonist inhibits TNF-a.

The method maybe provided wherein the HAAMMP is administered in an amount effective to inhibit MMPs present in the nucleus pulposus and to help arrest degradation of an extracellular matrix.

The method maybe provided wherein the TIMP is a recombinant TIMP.

The method maybe provided wherein the TIMP is present in an autologous concentrated form.

The method maybe provided wherein the anti-aggrecanase is present in an autologous concentrated form.

The method maybe provided wherein the anti-aggrecanase is a recombinant anti-aggrecanase.

The method maybe provided wherein the concentration of HAAMMP in the formulation is at least 100 mg/ml.

The method maybe provided wherein the formulation further comprises a sustained release device.

The method maybe provided wherein the sustained release device comprises a hydrogel.

The method maybe provided wherein the sustained release device provides controlled release.

The method maybe provided wherein the sustained release device provides continuous release.

The method maybe provided wherein the sustained release device provides intermittent release.

The method maybe provided wherein the sustained release device comprises a biosensor.

The method maybe provided wherein the sustained release device comprises microspheres.

The method maybe provided wherein the HAAMMP is predominantly released from the sustained delivery device by diffusion through the sustained delivery device or by biodegradation of the sustained delivery device.

The method maybe provided wherein the sustained release device comprises an inflammatory-responsive delivery system.

The method maybe provided wherein the HAAMMP is present in the formulation in a maximum amount of about 0.5 mg.

The method maybe provided wherein the formulation further comprises a growth factor present in an amount effective to repair disc tissue.

The method maybe provided wherein the growth factor is a TGF-P.

The method maybe provided wherein the growth factor is provided by platelet concentrate.

The method maybe provided wherein the concentration of cycline compound in the formulation is less than about 100 mg/ml.

The method maybe provided wherein the formulation is injected into the nucleus pulposus.

The method maybe provided wherein the formulation is injected into the annulus fibrosus.

The method maybe provided wherein the needle has a maximum gauge of 24 gauge.

The method maybe provided wherein the administration comprises providing the formulation in a patch attached to an outer wall of the annulus fibrosus.

The method maybe provided wherein the administration comprises providing the formulation in a depot closely adjacent to an outer wall of the annulus fibrosus.

The method maybe provided wherein the adminstration comprises providing the formulation in a depot closely adjacent an endplate of an adjacent vertebral body.

The method maybe provided wherein the degenerating disc is an intact disc.

The method maybe provided wherein the degenerating disc is a ruptured disc.

The method maybe provided wherein the degenerating disc is delaminated.

The method maybe provided wherein the degenerating disc has fissures.

The method maybe provided wherein the HAAMMP is predominantly released from the sustained delivery device by diffusion of the HAAMMP through a sustained delivery device.

The method maybe provided wherein the sustained delivery device is a polymer.

The method maybe provided wherein the HAAMMP is predominantly released from the sustained delivery device by biodegradation of the sustained delivery device.

The method maybe provided wherein the HAAMMP is a specific antagonist of a collagenase MMP.

The method maybe provided wherein the HAAMMP is a specific antagonist of stromelysin MMP.

The method maybe provided wherein the HAAMMP is a specific antagonist of gelatinase MMP.

The method maybe provided wherein the HAAMMP is a specific antagonist of a membrane MMP.

The method maybe provided wherein the HAAMMP is a specific antagonist of MMP-2.

The method maybe provided wherein the HAAMMP is a specific antagonist of MMP-3.

The method maybe provided wherein the HAAMMP is a specific antagonist of MMP-8.

The method maybe provided wherein the concentration of the cycline compound is at least about 20 mg/ml.

The method maybe provided wherein the additional therapeutic agent is a growth factor.

The method maybe provided wherein the cycline compound is administered in an amount effective to inhibit TNF-a present in the nucleus pulposus and thereby arrest degradation of an extracellular matrix.

The method maybe provided wherein the administration comprises providing the formulation in a patch attached to an outer wall of the annulus fibrosus.

The formulation wherein the HAAMMP is selected from the group consisting of TIMP-I and TIMP-2.

The formulation wherein the HAAMMP is an anti-aggrecanase.

The method maybe provided wherein the p38 kinase inhibitor is substantially water insoluble.

The method maybe provided wherein the p38 kinase is administered in a dosage to produce a local tissue concentration of between about 5 ug/kg and 50 Rg/kg.

The method maybe provided wherein the p38 kinase inhibitor is water soluble.

The method maybe provided wherein the 1-aryl-2-pyridinyl heterocycle is selected from the group consisting of : f) 4, 5 substituted imidazole; g) 1,4, 5 substituted imidizole ; h) 2,4, 5 substituted imidizole; i) 1,2, 4,5 substituted imidizole ; and j) non-imidizole 5-membered ring heterocycle.

The method maybe provided wherein the p38 kinase inhibitor has at least 3 cyclic groups.

The method maybe provided wherein the p38 kinase inhibitor is present in the formulation in an amount of at least about 100 mg/ml.

The method maybe provided wherein the p38 kinase inhibitor is present in the formulation in an amount of no more than about 0.5 mg.

The method maybe provided wherein the formulation further comprises glycosaminoglycans.

The method maybe provided wherein the additional therapeutic agent is plasmid DNA.

The method maybe provided wherein the proinflammatory protein is an interleukin.

The method maybe provided wherein the predetermined level for the interleukin is at least 100 pg/ml.

The method maybe provided wherein the predetermined level for the interleukin-6 is at least 100 pg/ml.

The method maybe provided wherein the predetermined level for the interleukin-6 is at least 250 pg/ml.

The method maybe provided wherein the predetermined level for the interleukin-8 is at least 500 pg/ml.

The method maybe provided wherein the proinflammatory protein is PGE2.

The method maybe provided wherein the predetermined level for PGE2 is at least 1000 pg/ml.

The method maybe provided wherein the proinflammatory protein is TNF-a.

The method maybe provided wherein the predetermined level for TNF-a is at least 20 pg/ml.

The method maybe provided wherein the predetermined level for TNF-a is at least 30 pg/ml.

The method maybe provided wherein the predetermined level for TNF-a is at least 1000 pg/disc.

The method maybe provided wherein the high specificity antagonist of NO synthase is selected from the group consisting of N-iminoethyl-L-lysine (L- NIL), and NG-monomethyl-L-arginine.

The method maybe provided wherein the high specificity anti-proliferative agent is rapamycin.

The method maybe provided wherein the rapamycin is provided in an about 0.1 to about 10 dose.

The method maybe provided wherein the high specificity anti-proliferative agent is a cdk inhibitor.

The method maybe provided wherein the cdk inhibitor is provided in an about 0.1 to about 10 uM dose.

The method maybe provided wherein the anti-apoptotic agent is selected from the group consisting of EPO, erythropoetin mimetic peptides, EPO mimetic antibody fusion proteins, IGF-I, IGF-II, and caspase inhibitors.

The method maybe provided wherein the formulation further comprises a buffer.

The method maybe provided wherein the cycline compound is administered in an amount effective to inhibit an MMP present in the nucleus pulposus and thereby arrest degradation of an extracellular matrix.

The method maybe provided wherein the cycline compound is administered in an amount effective to inhibit TNF-a present in the nucleus pulposus and thereby reduce pain.

The method maybe provided wherein the additional therapeutic agent comprises a high specificity antagonist of a pro-inflammatory interleukin.

The method maybe provided wherein the interleukin is IL-I β, interleukin is IL- 2, interleukin is IL-6, interleukin is IL-8, interleukin is IL-12, and interleukin is IL-19.

The method maybe provided wherein the additional therapeutic agent is a high specificity antagonist of TNF-a.

The method maybe provided wherein the high specificity antagonist of TNF-a is an inhibitor of soluble TNF-a.

The method maybe provided wherein the high specificity antagonist of TNF-a is an inhibitor of TNF-a synthesis.

The method maybe provided wherein the high specificity antagonist of TNF-a is an inhibitor of a natural receptor of TNF-a.

The method maybe provided wherein the highly specific antagonist of TNF-a is an inhibitor of p38 kinase.

The method maybe provided wherein the highly specific antagonist of TNF-a is an inhibitor of p38 kinase that is substantially water insoluble.

The method maybe provided wherein the additional therapeutic agent is a high specificity antagonist of NO synthase.

The method maybe provided wherein the high specificity antagonist is L-NIL.

The method maybe provided wherein the high specificity antagonist is NG-monomethyl-L-arginine.

The method maybe provided wherein the additional therapeutic agent is a high specificity antagonist of PLA2- 127. The method of claim 33, wherein the additional therapeutic agent is a high specificity anti-proliferative agent.

The method maybe provided wherein the high specificity anti-proliferative agent comprises rapamycin.

The method maybe provided wherein the high specificity anti-proliferative agent comprises a cdk inhibitor.

The method maybe provided wherein the high specificity anti-proliferative agent comprises a statin.

The method maybe provided wherein the high specificity anti-proliferative agent comprises an anti-oxidant.

The method maybe provided wherein the anti-oxidant comprises a super oxide dismutase.

The method maybe provided wherein the additional therapeutic agent is a highly specific apoptosis inhibitor selected from the group consisting of an EPO mimetic peptide and an EPO mimetibody.

The method maybe provided wherein the additional therapeutic agent is a highly specific apoptosis inhibitor selected from the group consisting of IGF-I and IGF-11.

The method maybe provided wherein the antagonist is a monoclonal antibody.

The method maybe provided wherein the monoclonal antibody is infliximab.

The method maybe provided wherein the antagonist is etanercept.

The method maybe provided wherein the antagonist binds a membrane-bound cytokine.

The method maybe provided wherein the antagonist binds a soluble cytokine.

The method maybe provided wherein the sustained release device comprises macrospheres.

### DETAILED DESCRIPTION OF THE INVENTION

For the purposes of the present invention, the terms"inhibitor"and "antagonist"are used interchangeably. A protein may be inhibited at the synthesis level, at the translation level, by shedding, by antibodies, or by soluble receptors. The term"patient"refers to a human having a degenerating disc. As used herein, antagonists include, for example, inhibitors of substances from a disc or present in a disc in patients with DDD. For example, the substances can be cytokines or MMPs. The antagonists can include, for example, a high specificity cytokine antagonist, a high affinity anti-matrix metalloproteinase (HAAMMP), a p38 kinase inhibitor (i. e., an antagonist of p3 8 kinase), e. g. , a high specificity antagonist of p38 kinase, and a cycline compound.

For the purposes of the present invention"transdiscal administration" includes, but is not limited to:
a) injecting a formulation into the nucleus pulposus of a degenerating disc, such as a relatively intact degenerating disc;
b) injecting a formulation into the annulus fibrosus of a degenerating disc, such as a relatively intact degenerating disc;
c) providing a formulation in a patch attached to an outer wall of the annulus fibrosus;
d) providing a formulation in a depot at a location outside but closely adjacent to an outer wall of the annulus fibrosus ("trans-annular administration"); and
e) providing a formulation in a depot at a location outside but closely adjacent to an endplate of an adjacent vertebral body (hereinafter, "trans-endplate administration".

Because DDD is a continuous process, the degenerating disc to which the therapeutic drug is administered may be in any one of a number of degenerative states. Accordingly, the degenerating disc may be an intact disc. The degenerating disc may be a herniated disc (*i*.*e*., wherein a portion of the annulus fibrosus has a bulge). The degenerating disc may be a ruptured disc (*i*.*e*., wherein the annulus fibrosus has ruptured and the bulk nucleus pulposus has exuded). The degenerating disc may be delaminated (*i*.*e*., wherein adjacent layers of the annulus fibrosus have separated). The degenerating disc may have fissures (*i*.*e*., wherein the annulus fibrosus has fine cracks or tears through which selected molecules from the nucleus pulposus can leak). In all of these degenerative states, the extra-cellular matrix of either the annulus fibrosus or nucleus pulposus is also degrading.

In one embodiment, the present invention is directed to providing directly through a diseased intervertebral disc at least one antagonist capable of specifically inhibiting a substance present in the disc, wherein the antagonist is selected from the group consisting of: a) a high specificity cytokine antagonist, b) a high affinity anti-matrix metalloproteinase (HAAMMP), c) an antagonist of p38 kinase, or d) a cycline compound.

US Published Patent Application No. US 2003/0039651 ("Olmarker I") teaches a therapeutic treatment of nerve disorders comprising administration of a therapeutically effective dosage of compounds, including inhibitors of MMPs, at least two substances selected from the group consisting of TNF inhibitors (both specific and non-specific), IL-1 inhibitors, IL-6 inhibitors, IL-8 inhibitors, FAS inhibitors, FAS ligand inhibitors, and IFN-gamma inhibitors.

In the examples of Olmarker I, it is taught that the therapeutic compounds are to be administered through systemic pathways. In particular, Olmarker I teaches that "the major contribution of TNF-alpha may be derived from recruited, aggregated and maybe even extravasated leukocytes, and that successful pharmacologic block may be achieved only by systemic treatment." Of note, Olmarker I appears to discourage the local addition of at least one therapeutic compound (doxycycline) to an autotransplanted nucleus pulposus to be applied to a spinal cord.

PCT Published Patent Application No. WO 02/100387 ("Olmarker II") teaches the prevention of neovascularization and/or neo-innervation of intervertebral discs by the administration of anti-angiogenic substances. Again, however, Olmarker II teaches systemic administration of these therapeutic agents.

U.S. Patent No. 6,419,944 ("Tobinick") discloses treating herniated discs with cytokine antagonists. Tobinick teaches that local administration involves a subcutaneous injection near the spinal cord. Accordingly, Tobinick does not teach a procedure involving a sustained delivery of a drug for the treatment of DDD, nor directly administering a drug into the disc.

U.S. Published Patent Application No. 2003/0049256 ("Tobinick II") discloses that injection of such therapeutic molecules to the anatomic area adjacent to the spine is accomplished by interspinous injection, and preferably is accomplished by injection through the skin in the anatomic area between two adjacent spinous processes of the vertebral column.

Tobinick II further teaches that the therapeutic compounds may be administered by interspinous injection in the human and that the dosage level is in the range of 1 mg to 300 mg per dose, with dosage intervals as short as two days. Tobinick II further discloses that other therapeutic compounds are administered in a therapeutically effective dose, which will generally be 10 mg to 200 mg per dose, and their dosage interval will be as short as once daily.

Tobinick, Swiss Med. Weekly, 133: 170-7 (2003) ("Tobinick III") teaches perispinal and epidural administration of TNF inhibitors.

Karppinen, Spine, 28(8): 750-4 (2003), teaches intravenously injecting or orally administering infliximab into patients suffering from sciatica.

As with Tobinick I and II, Karppinen does not teach a procedure involving a sustained delivery of a drug for the treatment of DDD, nor directly administering a drug into the disc space.

U.S. Patent No. 6,352,557 (Ferree) teaches adding therapeutic substances such as anti-inflammatory medications to morselized extra-cellular matrix, and injecting that combination into an intervertebral disc. However many anti-inflammatory agents are non-specific and, therefore, may produce unwanted side effects upon other cells, proteins and tissue. In addition, the pain-reducing effect of these agents is typically only temporary. Lastly, these agents typically only relieve pain, and are neither curative nor restorative.

Alini, Eur. Spine J., 11(Supp.2): S215-220 (2002), teaches therapies for early stage DDD, including injection of inhibitors of proteolytic enzymes or biological factors that stimulate cell metabolic activity (*i*.*e*., growth factors) in order to slow down the degenerative process. Inhibitors of proteolytic enzymes constitute a broad class of compounds, including i) inhibitors of proteolytic enzyme synthesis and ii) inhibitors of proteolytic enzyme activity. Alini I does not specify any desired types of inhibitors of proteolytic enzymes.

U.S. Published Patent Application US 2002/0026244 ("Trieu") discloses an intervertebral disc nucleus comprising a hydrogel that may deliver desired pharmacological agents. Trieu teaches that these pharmacological agents may include growth factors such as TGF-β and anti-inflammatory drugs, including steroids. Trieu further teaches that these pharmacological agents may be dispersed within the hydrogel having an appropriate level of porosity to release the pharmacological agent at a desired rate. Trieu teaches that these agents may be released upon cyclic loading or upon resorption.

Goupille, Spine, 23(14): 1612-1626 (1998) identifies Tissue Inhibitors of MMPs ("TIMPs") as degraders of MMP activity. Goupille reports that TIMP-1 and TIMP-2 bind noncovalently to active MMPs in a 1:1 molar ratio and specifically inhibit their enzymatic activity. However, Goupille also identifies corticosteroids, retinoic acid, TGF-β, PGE1 and PGE2 as inhibitors of MMP synthesis; identifies α2-macroglobulin, hydroxamic acid, derivatives, tetracyclines and quinolones as inhibitors of MMP activity, and identifies bFGF, EGF, Retenoic acid, TGF-β, IL-6, IL-1 LIF, dexamethasone, phorbol ester, and synthetic Vitamin A analogs as stimulators of TIMPs. Moreover, as to administration route, Goupille explicitly identifies only the oral administration route.

Takegami, Spine, 27(12):1318-25 (2000) teaches that injecting TGF-β into the disc space results in enhanced replenishment of the extracellular matrix damaged by cytokines. Takegami further teaches that the half-life of a growth factor injected into the intervertebral disc can be expected to be longer than that injected into a synovial joint because the nucleus pulposus is surrounded by the fibrous structure of the annulus fibrosus, thus providing a confined environment. Diwan, Tissue Engineering in Orthopedic Surgery, 31 (3):453-464 (2000), reports on another Takegami paper that concluded that a delivery system allowing prolonged delivery (>3 days) would have to be used to obtain the observed effect of the growth factor.

Alini, Spine 28(5):446-54 (2003), discloses a cell seeded collagen-hyaluronan scaffold supplemented with growth factors such as TGF-β, bFGF, and IGF-1 for use in regenerating a nucleus pulposus.

Maeda et al. Spine 25(2):166-169 (2000) reports on the *in vitro* response to interleukin-1 receptor antagonist protein (IRAP) of rabbit annulus fibrosus exposed to IL-1. Maeda suggests that IRAP could be useful in inhibiting the degradation of the disc.

Yabuki, Spine, 26(8):870-5 (2001), teaches the use of an anti-TNF drug for the treatment of sciatica.

U.S. Patent No. 6,277,969 ("Le") discloses the use of anti-TNF antibodies for therapy of TNF-mediated pathologies. Le teaches parenteral administration of the antibodies.

Ariga, Spine, 28(14):1528-33 (2003), reports that the application of a p38 MAP kinase inhibitor to a culture of organ-cultured intervertebral discs increased the occurrence of apoptosis in endplate chondrocytes in the culture.

Olmarker, Spine, 26(8): 863-9 (2001) ("Olmarker I") and Aoki, Spine, 27(15): 1614-17 (2002) teach that TNF-α (TNF-alpha) appears to play a role in producing the pain associated with the nucleus pulposus contacting nerve roots of the spinal cord. Olmarker discourages the use of non-specific TNF-α inhibitors in spine-related drug therapies, and particularly those that have anti-biotic effects.

U.S. Published Patent Application No. U.S. 2003/0039651 ("Olmarker II") teaches a therapeutic treatment of nerve disorders comprising administration of a therapeutically effective dosage of a number of drugs, including cycline compounds. According to Olmarker, tetracycline are non-specific inhibitors of TNF-α.

In the examples of Olmarker II, Olmarker II further teaches that these drugs are to be administered through systemic pathways. In particular, Olmarker II teaches that "the major contribution of TNF-α may be derived from recruited, aggregated and maybe even extravasated leukocytes, and that successful pharmacologic block may be achieved only by systemic treatment. Of note, Olmarker II appears to discourage the local addition of doxycycline to an autotransplanted nucleus pulposus to be applied to a spinal cord.

PCT Published Patent Application No. WO 02/100387 ("Olmarker III") teaches the prevention of neovasculariation and/or neo-innervation of intervertebral discs by the administration of anti-angiogenic substances. Again, however, Olmarker III teaches systemic administration of these therapeutic agents.

U.S. Patent No. 6,419,944 ("Tobinick") discloses treating herniated discs with cytokine antagonists, including infliximab. However, Tobinick teaches that local administration involves a subcutaneous injection near the spinal cord. Accordingly, Tobinick does not teach a procedure involving a sustained delivery of a drug for the treatment of DDD, nor directly administering a cycline compound into the disc.

U.S. Published Patent Application No. 2003/0049256 ("Tobinick II") discloses that injection of such therapeutic molecules to the anatomic area adjacent to the spine is accomplished by interspinous injection, and preferably is accomplished by injection through the skin in the anatomic area between two adjacent spinous processes of the vertebral column.

Tobinick, Swiss Med. Weekly, 133:170-77 (2003) ("Tobinick III") teaches perispinal and epidural administration of specific TNF-α inhibitors.

Karppinen, Spine, 28(8): 750-4 (2003), teaches intravenously injecting or orally administering infliximab into patients suffering from sciatica.

As with Tobinick I and II, Karppinen does not teach a procedure involving a sustained delivery of a drug for the treatment of DDD, nor directly administering a cycline compound into the disc.

In sum, although investigators have generally taught the transdiscal administration of inhibitors of proteolytic enzymes, when investigators have specifically identified the administration of a cycline compound for spine-related therapy, the investigators appear to teach only the administration of certain antagonists (e.g., a cycline compound) to tissue outside the disc. Moreover, Olmarker II appears to discourage the administration of a cycline compound to a nucleus pulposus.

### HIGH AFFINITY ANTI-MATRIX METALLOPROTEASE (HAAMMP)

In one embodiment, the HAAMMP is administered in an amount effective to inhibit the specific action of MMPs released by disc cells during the degenerative process. In one embodiment, the HAAMMP is administered in an amount effective to inhibit MMPs present in the nucleus pulposus and thereby help arrest degradation of an extracellular matrix.

In one embodiment, the HAAMMP is recombinant. In one embodiment, the HAAMMP is present in an autologous form. The HAAMMP can be present, for example, in an autologous concentrated form. In some embodiments, the HAAMMP comprises a chelating group that binds tightly to the zinc component present in the active site of the MMP. Such HAAMMPs may be selected from the materials disclosed in Gordon, Clin. Exp. Rheumatol., 11(Supp 8): S91-4 (1993); and Johnson, J., Enzyme Inhib., 2:1-22 (1987).

In some embodiments, the HAAMMP is a natural inhibitor of MMPs, *e.g.*, a tissue inhibitor of MMPs (TIMP). In some embodiments, the TIMP is selected from the group consisting of TIMP-1 and TIMP-2. In some embodiments, the TIMP is autologous and is concentrated by filtration, centrifugation or by immuno-attachment processes. In other embodiments, the TIMP is manufactured recombinantly, and is preferably present in a concentration of, *e.g.,* at least 1000 times that found normally in the patient. In some embodiments, the TIMP can be obtained from a heterologous source.

In some embodiments, the HAAMMP is a specific inhibitor of aggrecanase (*i.e*., anti-aggrecanase). In one embodiment, the anti-aggrecanase is present in an autologous concentrated form. In one embodiment, the anti-aggrecanase is a recombinant anti-aggrecanase.

In some embodiments, the HAAMMP is a specific antagonist of a collagenase MMP. In some embodiments, the HAAMMP is a specific antagonist of a stromelysin MMP. In some embodiments, the HAAMMP is a specific antagonist of a gelatinase MMP. In some embodiments, the HAAMMP is a specific antagonist of a membrane MMP.

In some embodiments, the targeted MMP is selected from the group consisting MMP-2, MMP-3 and MMP-8. Targeting MMP-2 and/or MMP-3 is desirable because these MMPs are believed to degrade proteoglycans. Targeting MMP-8 is desirable because this MMP is believed to degrade aggrecans.

In some embodiments, when the antagonist is a HAAMMP, an additional therapeutic substance (i.e., agent) is a high specificity cytokine antagonist ("HSCA"). For example, the high affinity cytokine antagonist can be selected from the group consisting of an HSCA of TNF-α, and an HSCA of an interleukin. In one embodiment, at least one of the therapeutic agents is an antagonist of TNF-α. In one embodiment, another therapeutic agent is an antagonist of an interleukin.

Since many pro-inflammatory proteins play a role in disc degeneration, and many of the antagonists of the present invention are highly specific, it is further believed that injecting at least two of the highly specific antagonists of the present invention directly into the disc would be advantageous.

Therefore, in accordance with the present invention, there is provide a method of treating degenerative disc disease in an intervertebral disc having a nucleus pulposus, comprising administering a formulation comprising HAAMMP and at least two highly specific antagonists of pro-inflammatory cytokines selected from the group consisting of TNF-α, an interleukin (preferably, IL-1, Il-6 and IL-8), FAS, an FAS ligand, and IFN-gamma.

### CYTOKINE ANTAGONISTS

In one embodiment, the HSCA inhibits the action of a specific proinflammatory cytokine released by disc cells or by invading macrophages during the degenerative process.

In some embodiments, the antagonist is capable of specifically inhibiting a pro-inflammatory cytokine selected from the group consisting of TNF-α, an interleukin (preferably, IL-1, Il-6 and IL-8), FAS, an FAS ligand, and IFN-gamma. Such specific inhibitors include those identified on pages 5-18 of Olmarker II.

In some embodiments, the HSCA inhibits the cytokine by preventing its production. In some embodiments, the HSCA inhibits the cytokine by binding to a membrane-bound cytokine. In others, the HSCA inhibits the cytokine by binding to a solubilized, e.g. soluble, cytokine. In some embodiments, the HSCA inhibitor inhibits the cytokine by both binding to membrane bound cytokines and to solubilized cytokines. In some embodiments, the HSCA is a monoclonal antibody ("mAb"). The use of mAbs is highly desirable since they bind specifically to a certain target protein and to no other proteins. In some embodiments, the HSCA inhibits the cytokine by binding to a natural receptor of the target cytokine.

In some embodiments, the HSCA inhibits the cytokine by preventing its production. One example thereof is an inhibitor of p38 mitogen activated protein (MAP) kinase.

In some embodiments, the HSCA is a specific antagonist of TNF-α. In some embodiments, the TNF inhibitor inhibits the TNF by binding to membrane-bound TNF in order to prevent its release from membrane. In others, the TNF inhibitor inhibits the TNF by binding to solubilized TNF. One example thereof is etanercept (ENBREL^{®}, Amgen). In some embodiments, the TNF inhibitor inhibits the TNF by both binding to membrane bound TNF and to solubilized TNF. One example thereof is REMICADE^{®} infliximab. In some embodiments, the HSCA inhibits the cytokine (e.g., TNF-α) by binding to a natural receptor of the target cytokine. In some embodiments, the TNF-α inhibitor is an inhibitor of TNF-α synthesis.

Preferred TNF antagonists include, but are not limited to, the following: etanercept (ENBREL^{®}, Amgen); infliximab (REMICADE^{®}-Johnson and Johnson); D2E7, a human anti-TNF monoclonal antibody (Knoll Pharmaceuticals, Abbott Laboratories); CDP 571 (a humanized anti-TNF IgG4 antibody); CDP 870 (an anti-TNF alpha humanized monoclonal antibody fragment), both from Celltech; soluble TNF receptor Type 1 (Amgen); pegylated soluble TNF receptor Type I (PEGs TNF-R1) (Amgen); and onercept, a recombinant TNF binding protein (r-TBP-1) (Serono).

TNF antagonists suitable for compositions, combination therapy, coadministration, devices and/or methods of the present invention (optionally further comprising at least one antibody, specified portion and/or variant thereof, of the present invention), include, but are not limited to, anti-TNF antibodies (e.g., at least one TNF antagonist (e.g., but not limited to, a TNF chemical or protein antagonist, TNF monoclonal or polyclonal antibody or fragment, a soluble TNF receptor (e.g., p55, p70 or p85) or fragment, fusion polypeptides thereof, or a small molecule TNF antagonist, e.g., TNF binding protein I or II (TBP-1 or TBP-II), nerelimonmab, REMICADE^{®} infliximab, enteracept (ENBREL^{®}), adalimulab (HUMIRA™), CDP-571, CDP-870, afelimomab, lenercept, and the like), antigen-binding fragments thereof, and receptor molecules which bind specifically to TNF; compounds which prevent and/or inhibit TNF synthesis, TNF release or its action on target cells, such as thalidomide, tenidap, phosphodiesterase inhibitors (e.g. pentoxifylline and rolipram), A2b adenosine receptor agonists and A2b adenosine receptor enhancers; compounds which prevent and/or inhibit TNF receptor signalling, such as mitogen activated protein (MAP) kinase inhibitors; compounds which block and/or inhibit membrane TNF cleavage, such as metalloproteinase inhibitors; compounds which block and/or inhibit TNF activity, such as angiotensin converting enzyme (ACE) inhibitors (e.g., captopril); and compounds which block and/or inhibit TNF production and/or synthesis, such as MAP kinase inhibitors.

As used herein, a "tumor necrosis factor antibody," "TNF antibody," "TNFα antibody," or fragment and the like decreases, blocks, inhibits, abrogates or interferes with TNFα activity *in vitro, in situ* and/or preferably *in vivo.* For example, a suitable TNF human antibody of the present invention can bind TNFα and includes anti-TNF antibodies, antigen-binding fragments thereof, and specified mutants or domains thereof that bind specifically to TNF-alpha (TNFα). A suitable TNF antibody or fragment can also decrease block, abrogate, interfere, prevent and/or inhibit TNF RNA, DNA or protein synthesis, TNF release, TNF receptor signaling, membrane TNF cleavage, TNF activity, TNF production and/or synthesis.

Chimeric antibody cA2 consists of the antigen binding variable region of the high-specificity neutralizing mouse anti-human TNFα IgG1 antibody, designated A2, and the constant regions of a human IgG1, kappa immunoglobulin. The human IgG1 Fc region improves allogeneic antibody effector function, increases the circulating serum half-life and decreases the immunogenicity of the antibody. The avidity and epitope specificity of the chimeric antibody cA2 is derived from the variable region of the murine antibody A2. In a particular embodiment, a preferred source for nucleic acids encoding the variable region of the murine antibody A2 is the A2 hybridoma cell line.

Chimeric A2 (cA2) neutralizes the cytotoxic effect of both natural and recombinant human TNFα in a dose dependent manner. From binding assays of chimeric antibody cA2 and recombinant human TNFα, the specificity constant of chimeric antibody cA2 was calculated to be 1.04x1010M-1. Preferred methods for determining monoclonal antibody specificity and specificity by competitive inhibition can be found in Harlow et al., Antibodies: A Laboratory Manual, Cold Spring Harbor Laboratory Press, Cold Spring Harbor, New York (1988); Colligan et al., eds., Current Protocols in Immunology, Greene Publishing Assoc. and Wiley Interscience, New York, (1992-2000); Kozbor et al., Immunol. Today, 4:72-79 (1983); Ausubel et al., eds. Current Protocols in Molecular Biology, Wiley Interscience, New York (1987-2000); and Muller, Meth. Enzymol., 92:589-601 (1983), which references are entirely incorporated herein by reference.

In a particular embodiment, murine monoclonal antibody A2 is produced by a cell line designated c134A. Chimeric antibody cA2 is produced by a cell line designated c168A.

Additional examples of monoclonal anti-TNF antibodies that can be used in the present invention are described in the art (see, e.g., U.S. Patent No. 5,231,024; Möller, A. et al., Cytokine 2(3):162-169 (1990); U.S. Application No. 07/943,852 (filed September 11, 1992); Rathjen et al., International Publication No. WO 91/02078 (published February 21, 1991); Rubin et al., EPO Patent Publication No. 0 218 868 (published April 22, 1987); Yone et al., EPO Patent Publication No. 0 288 088 (October 26, 1988); Liang, et al., Biochem. Biophys. Res. Comm. 137:847-854 (1986); Meager, et al., Hybridoma 6:305-311 (1987); Fendly et al., Hybridoma 6:359-369 (1987); Bringman, et al., Hybridoma 6:489-507 (1987); and Hirai, et al., J. Immunol. Meth. 96:57-62 (1987) which references are entirely incorporated herein by reference.

Preferred TNF receptor molecules useful in the present invention are those that bind TNFα with high specificity (*see, e.g.,* Feldmann et al., International Publication No. WO 92/07076 (published April 30, 1992); Schall et al., Cell, 61:361-370 (1990); and Loetscher et al., Cell 61:351-359 (1990), which references are entirely incorporated herein by reference and, optionally, possess low immunogenicity. In particular, the 55 kDa (p55 TNF-R) and the 75 kDa (p75 TNF-R) TNF cell surface receptors are useful in the present invention. Truncated forms of these receptors, comprising the extracellular domains (ECD) of the receptors or functional portions thereof (see, e.g., Corcoran et al., Eur. J. Biochem. 223:831-840 (1994)), are also useful in the present invention. Truncated forms of the TNF receptors, comprising the ECD, have been detected in urine and serum as 30 kDa and 40 kDa TNF inhibitory binding proteins (Engelmann, H. et al., J. Biol. Chem. 265:1531-1536 (1990)). TNF receptor multimeric molecules and TNF immunoreceptor fusion molecules, and derivatives and fragments or portions thereof, are additional examples of TNF receptor molecules which are useful in the methods and compositions of the present invention. The TNF receptor molecules which can be used in the invention are characterized by their ability to treat patients for extended periods with good to excellent alleviation of symptoms and low toxicity. Low immunogenicity and/or high specificity, as well as other undefined properties, can contribute to the therapeutic results achieved.

TNF receptor multimeric molecules useful in the present invention comprise all or a functional portion of the ECD of two or more TNF receptors linked via one or more polypeptide linkers or other nonpeptide linkers, such as polyethylene glycol (PEG). The multimeric molecules can further comprise a signal peptide of a secreted protein to direct expression of the multimeric molecule. These multimeric molecules and methods for their production have been described in U.S. Application No. 08/437,533 (filed May 9, 1995) the content of which is entirely incorporated herein by reference.

TNF immunoreceptor fusion molecules useful in the methods and compositions of the present invention comprise at least one portion of one or more immunoglobulin molecules and all or a functional portion of one or more TNF receptors. These immunoreceptor fusion molecules can be assembled as monomers, or hetero- or homo-multimers. The immunoreceptor fusion molecules can also be monovalent or multivalent. An example of such a TNF immunoreceptor fusion molecule is TNF receptor/IgG fusion protein. TNF immunoreceptor fusion molecules and methods for their production have been described in the art (Lesslauer et al., Eur. J. Immunol. 21:2883-2886 (1991); Ashkenazi et al., Proc. Natl. Acad. Sci. USA 88:10535-10539 (1991); Peppel et al., J. Exp. Med. 174:1483-1489 (1991); Kolls et al., Proc. Natl. Acad. Sci. USA 91:215-219 (1994); Butler et al., Cytokine 6(6):616-623 (1994); Baker et al., Eur. J. Immunol. 24:2040-2048 (1994); Beutler et al., U.S. Patent No. 5,447,851; and U.S. Application No. 08/442,133 (filed May 16, 1995). Methods for producing immunoreceptor fusion molecules can also be found in Capon et al., U.S. Patent No. 5,116,964; Capon et al., U.S. Patent No. 5,225,538; and Capon et al., Nature 337:525-531 (1989), which references are entirely incorporated herein by reference.

A functional equivalent, derivative, fragment or region of a TNF receptor molecule refers to the portion of the TNF receptor molecule, or the portion of the TNF receptor molecule sequence which encodes the TNF receptor molecule, that is of sufficient size and sequences to functionally resemble TNF receptor molecules that can be used in the present invention (e.g., bind TNFα with high specificity and possess low immunogenicity). A functional equivalent of a TNF receptor molecule also includes modified TNF receptor molecules that functionally resemble TNF receptor molecules that can be used in the present invention (e.g., bind TNFα with high specificity and possess low immunogenicity). For example, a functional equivalent of a TNF receptor molecule can contain a "SILENT" codon or one or more amino acid substitutions, deletions or additions (e.g., substitution of one acidic amino acid for another acidic amino acid; or substitution of one codon encoding the same or different hydrophobic amino acid for another codon encoding a hydrophobic amino acid). See Ausubel et al., Current Protocols in Molecular Biology, Greene Publishing Assoc. and Wiley-Interscience, New York (1987-2003).

In some embodiments, the monoclonal antibody that inhibits TNF-α is selected from the group consisting of monoclonal rodent-human antibodies, rodent antibodies, human antibodies or any portions thereof, having at least one antigen binding region of an immunoglobulin variable region, which antibody binds TNF. Preferably, this monoclonal antibody is selected from the group of compounds disclosed in U.S. Patent No. 6,277,969. In some embodiments, the REMICADE^{®} infliximab is delivered in a formulation having an infliximab concentration of between about 30 mg/ml and about 60 mg/ml.

It is further believed that transdiscal administration of an effective amount of other antagonists of pro-inflammatory compounds would also help provide therapy to the patient having DDD. In many embodiments, the antagonist is an antagonist of an enzyme, such as an enzyme of a kinase.

It is further believed that transdiscal administration of an effective amount of an antagonist of p38 kinase would also help provide therapy to the patient having DDD. It is believed that the p38 kinase site regulates the production of TNF-α, IL-1 and COX-2 enzyme. Therefore, in accordance with another embodiment of the present invention, there is provided a method of treating degenerative disc disease in an intervertebral disc having a nucleus pulposus, comprising transdiscally administering an effective amount of a formulation comprising a high specificity antagonist of p38 kinase into an intervertebral disc.

Accordingly, in some embodiments, the antagonist, e.g., the highly specific (i.e., high specificity) antagonist, is an inhibitor of p38 MAP kinase, preferably, a small molecule inhibitor of p38 MAP kinase. The inhibition of p38 MAP kinase is believed to block production of both TNF-α and Il-2, both of which are proinflammatory cytokines. The small molecule inhibitors of p38 MAP kinase are very specific and potent (∼ nM). Without wishing to be tied to a theory, it is believed that inhibition of p38 should not block TGF signaling nor TGF activity.

It is further believed that p38 inhibitors may also block induction of some metalloproteinases, COX 2 and NO synthetase. It is further believed that p38 inhibitors do not inhibit interleukins involved in immune cell proliferation such as IL-2. Preferably, they are provided in a 10 nM to 10 uM dose. Some antagonists of p38 kinase are disclosed in Zhang, J. Biol. Chem., 272(20), May 16 1997 (13397-402); Pargellis, Nature Structural Biology, 9(4), April 2002268-272, and Chae, Bone, 28(1), 45-53 (January 2001), and in U.S. Patent No. 6,541,477 ("Goehring"), the specification of which is hereby incorporated by reference in its entirety. In one embodiment, the HSA of p38 kinase is administered in a dosage to produce a local tissue concentration of between about 5 ug/kg and about 50 ug/kg.

In some embodiments, the p38 kinase inhibitor is selected from the group consisting of:
a) diaryl imidizole;
b) N,N'-diaryl urea (developed by Bayer, Boehringer Ingelheim and Vertex);
c) N,N-diaryl urea (developed by Vertex);
d) benzophenone (developed by Leo Pharmaceuticals);
e) pyrazole ketone (developed by Hoffman-LaRoche);
f) indole amide (developed by GlaxoSmithKline and Scios);
g) diamides (developed by AstraZeneca);
h) quinazoline (developed by GlaxoSmithKline);
i) pyrimido [4,5-d]pyrimidinone (developed by GlaxoSmithKline and Hoffman LaRoche); and
j) pyridylamino-quinazolines (developed by Scios).

Members of this group are described, for example, in Zhang *et al., supra,* Pargellis *et al., supra,* Chae *et al., supra,* and Cirillo et al., Current Topics in Medicinal Chemistry, 2: 1021-1035 (2002), Boehm et al., Exp. Opin, Ther. Patents, 10(1):25-38 (2000), and Lee et al., Immunopharmacology, 47: 185-2001 (2000).

In some embodiments, the p38 kinase inhibitor is selected from the group consisting of:
a) SK&F 86002;
b) SB 203580;
c) L-167307;
d) HEP 689;
e) SB220025;
f) VX-745;
g) SU4984;
h) RWJ 68354;
i) ZM336372;
j) PD098059;
k) SB235699; and
l) SB220025.

In some embodiments, the p38 kinase inhibitor is characterized as a 1-aryl-2-pyridinyl heterocycle. In some embodiments, the 1-aryl-2-pyridinyl heterocycle is selected from the group consisting of:
a) 4,5 substituted imidazole,
b) 1,4,5 substitutued imidizole;
c) 2,4,5 substututued imidizole;
d) 1,2,4,5 substituted imidizole; and
e) non-imidizole 5-membered ring heterocycle.

In some embodiments, the p38 kinase inhibitor has at least 3 cyclic groups.

In some embodiments, the p38 kinase inhibitor is readily soluble in water (water soluble). In some embodiments, the p38 kinase inhibitor is a substantially water insoluble molecule. The substantially water insoluble p38 inhibitor may be advantageous in that, if injected into the nucleus pulposus, it will remain in the nucleus pulposus as a solid and only slightly solubize over time, thereby providing sustained release.

In one embodiment, the formulation further comprises the p38 kinase inhibitor and glycosaminoglycans.

In some embodiments, the HSCA is a specific antagonist of an interleukin. Preferably, the target interleukin is selected from the group consisting IL-1, IL-2, IL-6 and IL-8, and IL-12. Preferred antagonists include but are not limited to Kineretg (recombinant IL 1-RA, Amgen), IL1-Receptor Type 2 (Amgen) and IL-1 Trap (Regeneron).

It is further believed that the present invention can also be used to prevent degeneration of an intervertebral disc in a human individual, namely, by following a procedure comprising:
a) determining a genetic profile of the individual;
b) comparing the profile of the individual against a pre-determined genetic profile level of at-risk humans;
c) determining that the individual is an at-risk patient; and
d) injecting an antagonist into a disc of the individual.

### CYCLINE COMPOUNDS

In one aspect, the present invention is directed to providing a cycline compound directly to (*e*.*g*., through) a diseased intervertebral disc. In one embodiment, there is provided a method of treating degenerative disc disease in an intervertebral disc having a nucleus pulposus, comprising transdiscally administering a formulation comprising a cycline compound into the intervertebral disc. In another embodiment, the formulation further comprises a buffer.

In one embodiment, the cycline compound is administered in an amount effective to inhibit the action of a pro-inflammatory cytokine (such as TNF-α) or a matrix metalloproteinase (MMP). These cytokines can be present in the nucleus pulposus. In one embodiment, the cycline compound is administered in an amount effective to inhibit the action of an MMP released by disc cells during the degenerative process. In one embodiment, the cycline compound is administered in an amount effective to both a) inhibit a specific pro-inflammatory cytokine (such as TNF-α), and b) inhibit an ECM-degrading MMP released by local cells during the degenerative process. In one embodiment, the cycline compound is administered in an amount effective to inhibit an MMP (e.g., MMP present in the nucleus pulposus) and thereby arrest degradation of an extracellular matrix. In one embodiment, the cycline compound is administered in an amount effective to inhibit TNF-α (e.g., TNF-α present in the nucleus pulposus) and thereby arrest degradation of an extracellular matrix and/or reduce pain.

In some embodiments, the cycline compound is selected from the group consisting of doxycycline, lymecycline, oxicycline compound, tetracycline, minocycline, chemically modified tetracycline (CMT) and KB-R7785. Preferably, doxycycline or minocycline is selected.

In some embodiments, the antagonist (e.g., a cycline compound) is administered with an additional therapeutic agent. In one embodiment, the additional therapeutic agent comprises an antagonist of a pro-inflammatory interleukin, interleukin is IL-1β, interleukin is IL-2, interleukin is IL-6, interleukin is IL-8, interleukin is IL-12 and interleukin is IL-19.

In one embodiment, the additional therapeutic agent is an antagonist of TNF-α. In one embodiment, the antagonist of TNF-α is an inhibitor of soluble TNF-α, TNF-α synthesis, a natural receptor of TNF-α, p38 kinase, and/or p38 kinase that is substantially water insoluble.

In some embodiments, the HSCA is a specific inhibitor *(e.g.,* a high specificity antagonist) of an interleukin, *e.g.*, a pro-inflammatory interleukin. Preferably, the target interleukin is selected from the group consisting IL-1, IL-1β, IL-2, IL-6, IL-8, IL-12 and IL-19. Preferred antagonists include, but are not limited to, Kineretg (recombinant IL 1-RA, Amgen), IL1-Receptor Type 2 (Amgen) and IL-1 Trap (Regeneron).

Since many pro-inflammatory proteins play a role in disc degeneration, and antagonists thereof are preferably highly specific, it is further believed that injecting at least two of the highly specific antagonists of the present invention directly into the disc would be advantageous.

Therefore, in accordance with the present invention, there is provided a method of treating degenerative disc disease in an intervertebral disc having a nucleus pulposus, comprising administering into an intervertebral disc a formulation comprising:
i) a cycline compound; and
ii) at least two highly specific antagonists of pro-inflammatory cytokines selected from the group consisting of TNF-α, an interleukin (*e.g.*, IL-1, Il-6 and IL-8), FAS, an FAS ligand, and IFN-gamma.

Cycline compounds inhibit TNF-α in a non-specific manner. TNF-α and other similar bioactive substances are first produced in an inactive form and transported to the cell membrane. Upon activation, the active part of the pro-TNF-α is cleaved and released. This process is called shedding and may be initiated by one or more enzymes. These enzymes all have in common a metal ion and are called matrix metalloproteinases (MMPs). Cycline compounds are known to bind to metal ions and will thereby inhibit the action of the MMP and subsequently the release of TNF-α and other pro-inflammatory cytokines in a non-specific manner.

### OTHER ANTAGONISTS AND AGENTS

It is further believed that transdiscal administration of an effective amount of an antagonist of the COX-2 enzyme would also help provide therapy to the patient having DDD. It is believed that the COX-2 enzyme is a regulator of the production of prostaglandins, which are involved both in inflammation and the generation of pain. Therefore, in accordance with another embodiment of the present invention, there is provided a method of treating degenerative disc disease in an intervertebral disc having a nucleus pulposus, comprising transdiscally administering an effective amount of a formulation comprising a high affinity antagonist of COX-2 enzyme into an intervertebral disc.

Typical antagonists of the COX-2 enzyme include Celecoxib (Searle), Rofecoxib (Merck); Meloxican (Boehringer Manheim); Nimesulide; diclofenac and Lodine.

It is further believed that transdiscal administration of an effective amount of an antagonist of the PLA₂ enzyme would also help provide therapy to the patient having DDD. It is believed that the PLA₂ enzyme is a regulator of the production of prostaglandin. Therefore, in accordance with another embodiment of the present invention, there is provided a method of treating degenerative disc disease in an intervertebral disc having a nucleus pulposus, comprising transdiscally administering an effective amount of a formulation comprising a high affinity antagonist of PLA₂ enzyme into an intervertebral disc. In some embodiments, the antagonist of PLA2 may be administered systemically.

At least one antagonist of PLA₂ is disclosed in Kawakami, Clin. Orthop., 351: 241-51 (1998).

It is further believed that transdiscal administration of an effective amount of an antagonist of the NO synthase enzyme would also help provide therapy to the patient having DDD. It is believed that the NO synthase enzyme regulates the production of NO, which is known to have pro-inflammatory effects. Therefore, in accordance with another embodiment of the present invention, there is provided a method of treating degenerative disc disease in an intervertebral disc having a nucleus pulposus, comprising transdiscally administering an effective amount of a formulation comprising a high affinity antagonist of NO synthase into an intervertebral disc. In some embodiments, the antagonists of NO synthase may be administered systemically.

Some antagonists of NO synthase are N-iminoethyl-L-lysine (L-NIL), and N^{G}-monomethyl-L-arginine.

It is further believed that transdiscal administration of an effective amount of a high specificity anti-oxidant would also help provide therapy to the patient having DDD. It is believed that oxidants degrade the nucleus pulposus extra-cellular matrix. Typical anti-oxidants include free radical scavengers and superoxide dismutase enzymes. Therefore, in accordance with another embodiment of the present invention, there is provided a method of treating degenerative disc disease in an intervertebral disc having a nucleus pulposus, comprising transdiscally administering an effective amount of a formulation comprising a high affinity antioxidant into an intervertebral disc. In some embodiments, the high specificity antioxidants may be administered systemically.

It is further believed that trandiscal administration of an effective amount of a high specificity anti-proliferative agent would also help provide therapy to the patient having DDD. It is believed that antiproliferative agents may have an effect on inflammation by affecting inflamed tissues which would limit the production of inflammatory cytokines. In some embodiments, the high specificity anti-proliferative is selected from the group consisting of a) rapamycin; b) an inhibitor of cycline dependent kinase 9 (cdk); and c) statins (such as MEVASTATIN^{™} and LOVASTATIN^{™}). In one embodiment, when rapamycin is selected, a dosage producing a local tissue concentration of between about 0.5 ug/kg and about 50 ug/kg is used.

Therefore, in accordance with another embodiment of the present invention, there is provided a method of treating degenerative disc disease in an intervertebral disc having a nucleus pulposus, comprising transdiscally administering an effective amount of a formulation comprising a high specificity anti-proliferative agent into an intervertebral disc.

Rapamycin is a potent inhibitor of downstream signaling of TOR (target of Rapamycin) proteins. As such, it is responsible for coordinating the balance between protein synthesis and protein degradation. It is believed that DDD is propagated by a loss of balance between extracellular matrix synthesis and degradation. Since TOR proteins regulate multiple metabolic pathways, rapamycin may stabilize the balance of the cycle. Rapamycin may also directly affect the proliferation and subsequent immune reaction of chondrocytes. In addition, degenerative chondrocytes demonstrate a low level of proliferative activity in contrast to normal chondrocytes which show no activity. This is thought to lead to chondrocyte clustering within the cartilage. Rapamycin could function to eliminate the atypical chondrocyte proliferation. In one embodiment, it is provided in an about 0.1 to about 10 µM dose.

Cdk inhibitors may directly affect the proliferation and subsequent immune reaction of chondrocytes. A cdk inhibitor may also have a direct effect on chondrocyte clustering which is known to be a characteristic degenerative event. Exemplary cdk inhibitors include flavopiridol, roscovitine, and compounds disclosed in PCT Patent Publication No. WO 02/057240 (Lin) and U.S. provisional patent application 60/257,703, the specifications of which are incorporated by reference herein in their entirety. In one embodiment, it is provided in a 1 to 10 µM dose.

The present invention is also directed to providing a highly specific anti-apoptosis molecule to the diseased joint. These molecules serve to protect against chondrocyte apoptosis. Preferred compounds include EPO, erythropoetin mimetic peptides, EPO mimetic antibody fusion protein, IGF-I , IGF-II, and caspase inhibitors.

Therefore, in accordance with another embodiment of the present invention, there is provided a method of treating degenerative disc disease in an intervertebral disc having a nucleus pulposus, comprising transdiscally administering an effective amount of a formulation comprising a high specificity anti-apoptotic agent into an intervertebral disc.

In addition, non-steroidal anti-inflammatory drugs (NSAIDs) may also be selected as the second therapeutic agent. In some embodiments, the NSAID is anabolic, and is preferably selected from the group consisting of TOLMETIN^{™} (available from Ortho-MacNeil), SUPROL^{™} (available from Johnson & Johnson), and Tiaprofenic acid, (available from Roussel Labs). Preferably, the anabolic NSAID is administered in a dosage sufficient to produce an initial local tissue concentration of between about 5 ug/kg and about 500 ug/kg. In some embodiments, the NSAID is a dual inhibitor of both the COX and LOX pathways, and is preferably TEPOXALIN^{™} (available from Johnson & Johnson).

In addition, anti-cathepsins may also be used in accordance with the present invention. It is believed that inhibition of these enzymes inhibits the breakdown of the extracellular matrix. Preferably, the antagonists inhibits a cathepsin selected from the group consisting of cathepsin B, cathepsin L and cathepsin K.

In some embodiments, the additional therapeutic agent is selected from the group consisting of:
i) a growth factor, and
ii) plasmid DNA.

### ADMINISTRATION TO PATIENTS WITH DEGENERATIVE DISC DISEASE

DDD involves the progressive degeneration of a disc in which many factors are involved. In many instances, simply providing a single dose or even a regimen over the space of a few days may not be sufficient to resolve the DDD. For example, if DDD were caused in part by mechanical instability in a functional spinal unit, then simply providing a one-time therapy for the transdiscal cells would likely only delay the onset of the DDD. Therefore, there is a need to provide a long-term drug therapy treatment of DDD that does not require multiple injections.

Because DDD is a continuous process and because it is believed that some of the cytokines of interest both produce pain and degrade the ECM when present within the nucleus pulposus, it is desirable for the antagonist to remain within the nucleus pulposus as long as possible in a pharmaceutically effective amount. The half-life of the antagonist within the nucleus pulposus will depend upon many factors, including the size of the antagonist and its charge. In general, the larger the molecular weight of the antagonist, the more likely it is to remain contained by the annulus fibrosus portion of the disc.

When using a short half-life antagonist, it would be desirable for a relatively large dose of the antagonist to be administered transdiscally (i.e., into the disc). In this condition, quick depletion of the antagonist would not cause the antagonist to fall below therapeutically effective levels in the disc until an extended period of time has elapsed.

Although a large dose of the antagonist would be desirable in such instances, injecting a critical volume of water can increase pressure in the nucleus pulposus. Nociceptors present on the inner wall of the annulus fibrosus react to this increased pressure and produce pain. In some cases, the added amount could be as little as one cc by volume to produce pain. Accordingly, if a dilute concentration of an antagonist is added to the nucleus pulposus to provide a large dose, the resulting pressure increase caused by this added volume could be sufficient to cause acute pain.

For example, if it were determined that about 100 mg of an antagonist was needed to therapeutically affect a nucleus pulposus, and that antagonist was provided in concentrations of from about 30 to about 60 mg/ml, then at least about 1.5 ml of the antagonist would need to be injected into the nucleus pulposus in order to provide the desired therapeutic effect. However, when injecting volumes into the nucleus pulposus, it is desirable that the volume of drug delivered be no more than about 1 ml, preferably no more than about 0.5 ml (*i.e.,* a maximum of 0.5 ml), more preferably between about 0.1 and about 0.3 ml. When injected in these smaller quantities, it is believed the added volume will not cause an appreciable pressure increase in the nucleus pulposus.

Accordingly, in some embodiments, the concentration of the antagonist in the administered formulation is at least about 100 mg/ml. When about 100 mg of the antagonist is needed to produce the desired therapeutic result, no more than about 1 ml of the drug need be injected. In some embodiments, the concentration of the antagonist in the administered drug is at least about 200 mg/ml. In this condition, no more than about 0.5 ml of the drug need be injected. In some embodiments, the concentration of the antagonist in the administered drug is at least about 500 mg/ml. In this condition, between about 0.03 to about 0.3 ml of the formulation need be injected.

In another example, if it were determined that about 2 mg of a cycline compound was needed to therapeutically affect a nucleus pulposus, and that cycline compound was provided in concentrations of about 2 mg/ml, then at least about 1 ml of the cycline compound would need to be injected into the nucleus pulposus in order to provide the desired therapeutic effect. However, when injecting volumes into the nucleus pulposus, it is desirable that the volume of drug delivered be less than 1 ml, preferably no more than 0.5 ml, more preferably between about 0.03 ml and about 0.3 ml. When injected in these smaller quantities, it is believed the added volume will not cause an appreciable pressure increase in the nucleus pulposus.

Accordingly, in some embodiments, the concentration of the cycline compound in the administered drug, *i.e*., formulation, (*i.e*., sustained delivery device) is at least about 20 mg/ml. In this condition, no more than 0.1 ml of the drug need be injected. Preferably, the concentration of the cycline compound in the administered drug is at least 50 mg/ml. In this condition, no more than about 0.05 ml *(i.e.,* a maximum of 0.05 ml) of the drug need be injected. In one embodiment, the concentration of cycline compound in the formulation is less than about 100 mg/ml.

In some embodiments, the antagonist is provided in a sustained release device (*i.e*., sustained delivery device). The sustained release device is adapted to remain within the disc for a prolonged period and slowly release the antagonist contained therein to the surrounding environment. This mode of delivery allows an antagonist to remain in therapeutically effective amounts within the disc for a prolonged period.

In some embodiments, the antagonist is predominantly released from the sustained delivery device by its diffusion through the sustained delivery device (*e.g.*, through a polymer). In others, the antagonist is predominantly released from the sustained delivery device by the biodegradation of the sustained delivery device *(e.g.,* biodegradation of a polymer).

In some embodiments, the sustained release device (i.e., sustained delivery device) comprises a bioresorbable material whose gradual erosion causes the gradual release of the antagonist to the disc environment. In some embodiments, the sustained release device comprises a bioresorbable polymer. In some embodiments, the bioresorbable polymer has a half-life of at least one month, more preferably at least two months, more preferably at least 6 months.

In some embodiments, the sustained delivery device comprises bioerodable macrospheres. The antagonist is preferably contained in a gelatin (or water or other solvent) within the capsule, and is released to the disc environment when the outer shell has been eroded. The device can include a plurality of capsules having outer shells of varying thickness, so that the sequential breakdown of the outer shells provides periodic release of the antagonist.

In some embodiments, the sustained release device provides controlled release. In some embodiments, it provides continuous release. In some embodiments, it provides intermittent release. In some embodiments, the sustained release device comprises a biosensor. Other release modes may also be used.

In some embodiments, the sustained delivery device comprises an inflammatory-responsive delivery system, such as one comprising bioerodable microspheres that are eroded by invading macrophages. This technology provides a high correspondence between physiologic inflammation of disc environment and the release of the antagonists into that environment. In one embodiment, the technology disclosed in Brown et al., Arthritis. Rheum. Dec., 41(12): 2185-95 (1998) is selected.

In some embodiments, the sustained delivery device comprises the devices disclosed in U.S. Patent No. 5,728,396 ("Peery") the specification of which is incorporated by reference in its entirety.
In some embodiments, the sustained delivery device comprises a liposomal delivery system, such as that disclosed in WO 03/000190. Liposomes are small spheres whose walls are layers of lipids with water. As they form, liposomes entrap water and any water soluble solutes that are present. Because of this entrapping ability, they are useful as delivery systems. For the purposes of the present invention, a preferred embodiment includes the use of a multilamellar vesicle, and any naturally occurring phospholipid, such as dipalmitoylphosphatidylcholine (DPPC).

A liposome may be a vesicle having at least one lipid bilayer surrounding an inner liquid phase (a lipid bilayer surrounding either a liquid core or a liquid phase dispersed between it and another lipid bilayer). The liposome may have various structures such as multilamellar (MLVs), unilamellar (ULVs) and paucilamellar (PLVs) vesicles. The resulting structure of the liposome is dependent, in part, on the choice of materials forming the hydrophobic phase and the manufacturing parameters, such as temperature and incubation time.

Some liposomes comprise at least one amphiphilic bilayer-forming substance. The therapeutic substances contained therein may be contained either within the lipid bilayer or the hydrophilic compartments of the liposome. The amphiphilic bilayer-forming substance comprises both a hydrophilic and a lipophilic group and is capable of forming, either alone or in combination with other lipids, the bilayer of a liposome. The lipid can have single or multiple lipophilic side chains being either saturated or unsaturated in nature and branched or linear in structure. The amphiphilic bilayer-forming substance can be a phospoholipid or a ceramide.

In some embodiments, the sustained delivery device comprises a plurality (e.g., at least one hundred) of water-containing chambers, each chamber containing the antagonist. A chamber is defined by bilayer lipid membranes comprising synthetic duplicates of naturally occurring lipids. Release of the drug can be controlled by varying at least one of the aqueous excipients, the lipid components, and the manufacturing parameters. Preferably, the formulation comprises no more than 10% lipid. In some embodiments, the DEPOFOAM^{™} technology of Skyepharma PLC (London, United Kingdom) is selected.

In some embodiments, the sustained delivery device comprises a delivery system disclosed in U.S. Patent No. 5,270,300 ("Hunziker"), the specification of which is incorporated by reference in its entirety.

In some embodiments, the sustained delivery device comprises the co-polymer poly-DL-lactide-co-glycolide (PLG). Preferably, the formulation is manufactured by combining the antagonist or additional therapeutic agent, the co-polymer and a solvent to form a droplet, and then evaporating the solvent to form a microsphere. The plurality of microspheres are then combined in a biocompatible diluent. Preferably, the antagonist or additional therapeutic agent is released from the co-polymer by its diffusion through and by the biodegradation of the co-polymer. In some embodiments hereof, the PROLEASE^{™} technology of Alkermes (located in Cambridge, MA) is selected.

Hydrogels can also be used to deliver the antagonist in a sustained release manner to the disc environment. A "hydrogel" is a substance formed when an organic polymer (natural or synthetic) is set or solidified to create a three-dimensional open-lattice structure that entraps molecules of water or other solution to form a gel. The solidification can occur, *e.g.*, by aggregation, coagulation, hydrophobic interactions, or cross-linking. The hydrogels employed in this invention can rapidly solidify to keep the HAAMMP at the application site, thereby eliminating undesired migration from the disc. The hydrogels are also biocompatible, *e.g.*, not toxic, to substances suspended in the hydrogel.

A "hydrogel-antagonist composition" is a suspension of a hydrogel containing desired antagonist. The hydrogel-antagonist composition forms a uniform distribution of antagonist with a well-defined and precisely controllable density. Moreover, the hydrogel can support very large densities of antagonist. In addition, the hydrogel allows diffusion of nutrients to, and waste products away from, the endplates, which promotes tissue growth.

Hydrogels suitable for use in the present invention include water-containing gels, *i.e*., polymers characterized by hydrophilicity and insolubility in water. See, for instance, "Hydrogels", pages 458-459 in Concise Encyclopedia of Polymer Science and Engineering, Eds. Mark et al., Wiley and Sons (1990), the disclosure of which is incorporated herein by reference in its entirety. Although their use is optional in the present invention, the inclusion of hydrogels is can be highly advantageous since they tend to possess a number of desirable qualities. By virtue of their hydrophilic, water-containing nature, hydrogels can assist with load bearing capabilities of the disc.

In one embodiment, the hydrogel is a fine, powdery synthetic hydrogel. Suitable hydrogels exhibit an optimal combination of such properties as compatibility with the matrix polymer of choice, and biocompatability. The hydrogel can include any one or more of the following: polysaccharides, proteins, polyphosphazenes, poly(oxyethylene)-poly(oxypropylene) block polymers, poly(oxyethylene)-poly(oxypropylene) block polymers of ethylene diamine, poly(acrylic acids), poly(methacrylic acids), copolymers of acrylic acid and methacrylic acid, poly(vinyl acetate), and sulfonated polymers.

In general, these polymers are at least partially soluble in aqueous solutions, *e.g.*, water, or aqueous alcohol solutions that have charged side groups, or a monovalent ionic salt thereof. There are many examples of polymers with acidic side groups that can be reacted with cations, *e.g.*, poly(phosphazenes), poly(acrylic acids), and poly(methacrylic acids). Examples of acidic groups include carboxylic acid groups, sulfonic acid groups, and halogenated (preferably fluorinated) alcohol groups. Examples of polymers with basic side groups that can react with anions are poly(vinyl amines), poly(vinyl pyridine), and poly(vinyl imidazole).

In some embodiments, the sustained delivery device includes a polymer selected from the group consisting of PLA, PGA, PCL and mixtures thereof.

If the half-life of the antagonist within the disc is relatively long, then it may be assumed that a relatively small dose of the antagonist can be administered into the disc. In this condition, the slow depletion of the antagonist would not cause the antagonist to fall below therapeutically effective levels in the disc until an extended period of time has elapsed.

In some embodiments in which antagonists have long half-lives within the disc space, the dose administered can be very small.

For example, if it is believed that an antagonist is effective when present in the range of about 1 to about 10 mg/kg or from about 1 to about 10 ppm (as is believed to be the case for the TNF antagonist REMICADE^{®} infliximab), and since a typical nucleus pulposus of a disc has a volume of about 3 ml (or 3 cc, or 3g), then only about 3 to about 30 ug of the antagonist need be administered to the disc in order to provide a long lasting effective amount of the antagonist. The formulation can be administered in an amount of less than 1 cc. As a point of reference, Tobinick discloses that at least 1 mg of cytokine antagonist should be administered perispinally in order to cure back pain. Similarly, Olmarker mixed 100 ml of a formulation comprising 1.11 mg/ml of a monoclonal antibody into 40 mg of an extracted nucleus pulposus, thereby producing a monoclonal antibody concentration of about 3 parts per thousand. The smaller amounts available by this route reduce the chances of deleterious side effects of the antagonist.

For example, if a clinician administered 0.3 ml of 60 mg/ml of an antagonist, such as REMICADE^{®} infliximab, into a 2.7 cc disc, this would produce an antagonist concentration in the disc of about 6 mg/ml, or 6 parts per thousand. Without wishing to be tied to a theory, if an antagonist (e.g., a HAAMMP) has the same half-life within a nucleus pulposus as it does when administered systemically (i.e, about 1 week), then the concentration of the antagonist would remain above about 10 ppm for about 9 weeks. Therefore, if another dose were needed, the clinician would only need to provide the second dose after about two months.

Therefore, in some embodiments, the antagonist is provided in a dose of less than about 1 mg, *e.g.*, less than about 0.5 mg, more preferably, less than about 0.1 mg, more preferably less than about 0.01 mg, more preferably less than about 0.001 mg. The smaller amounts available by this route reduce the chances of deleterious side effects of the antagonist. When the antagonist is a HSCA, then preferably, the HSCA provided in these smaller amounts is a TNF antagonist, more preferably it is REMICADE^{®} infliximab.

In some embodiments, the formulation of the present invention is administered directly into the disc through the outer wall of the annulus fibrosus. More preferably, the direct administration includes depositing the antagonist in the nucleus pulposus portion of the disc. In this condition, the fibrous nature of the annulus fibrosus that surrounds the nucleus pulposus will help keep the antagonist contained within the disc.

In some embodiments, the formulation of the present invention is injected into the disc through a small bore needle. In some embodiments, the needle has a bore of about 22 gauge or less, so that the possibilities of producing a herniation are mitigated. For example, the needle can have a bore of about 24 gauge or less, so that the possibilities of producing a herniation are even further mitigated.

If the volume of the direct injection of the formulation is sufficiently high so as to cause a concern of overpressurizing the nucleus pulposus, then it is preferred that at least a portion of the nucleus pulposus be removed prior to direct injection. In some embodiments, the volume of removed nucleus pulposus is substantially similar to the volume of the formulation to be injected. For example, the volume of removed nucleus pulposus can be within 80-120% of the volume of the formulation to be injected. In addition, this procedure has the added benefit of at least partially removing some degenerated disc from the patient.

In other embodiments, the formulation is delivered into the disc space through the endplate of an opposing vertebral body. This avenue eliminates the need to puncture the annulus fibrosus, and so eliminates the possibility of herniation.

Although the antagonist may therapeutically treat the disc by binding an MMP, thereby reducing pain and arresting degradation of the ECM, it is believed that at least some of these antagonists do not help repair the damage done by the MMP to the ECM.

In accordance with the present invention, there is provided a method of treating degenerative disc disease in an intervertebral disc having a nucleus pulposus, comprising:
a) transdiscally administering an antagonist into a degenerating disc;
   and

In another embodiment, there may be a need to provide a therapy that also helps repair the ECM.
b) transdiscally administering at least one additional therapeutic agent therefore, an additional therapeutic agent maybe administered in an amount effective to at least partially repair the disc (*i.e*., the disc tissue).

In accordance with one aspect of the invention, both the antagonist and at least one additional (e.g., second) therapeutic agent(s) are locally administered into the disc space. When the antagonist is HSCA, because the HSCA is specific, it does not interfere with the locally administered additional therapeutic agent, and so each agent may independently work to provide therapy to the diseased disc.

More than one additional therapeutic agent can be administered. For example, there can be third, fourth and fifth therapeutic agents.

In some embodiments, the antagonist and additional therapeutic agent(s) (*i.e.*, additional therapeutic substance) are administered simultaneously. In one embodiment, the antagonist and additional therapeutic agents are co-administered in a formulation comprising the antagonist and the additional therapeutic agent. In others, the antagonist(s) are administered first. In still others, the second therapeutic agent is administered first.

The additional therapeutic agent(s) can be one or more of the antagonists disclosed herein. For example, other compounds which may be added to the disc include, but are not limited to: vitamins and other nutritional supplements; hormones; glycoproteins; fibronectin; peptides and proteins; carbohydrates (both simple and/or complex); proteoglycans; oligonucleotides (sense and/or antisense DNA and/or RNA); bone morphogenetic proteins (BMPs); antibodies (for example, to infectious agents, tumors, drugs or hormones antiangiogenins; demineralized bone matrix (DBM); antibodies (for example, to infectious agents, tumors, or drugs); gene therapy reagents; and anti-cancer agents. If desired, substances such as pain killers and narcotics may also be admixed with the polymer for delivery and release to the disc space.

In some embodiments, growth factors are additional therapeutic agents. As used herein, the term "growth factors" encompasses any cellular product that modulates the growth or differentiation of other cells, particularly connective tissue progenitor cells. The growth factors that may be used in accordance with the present invention include, but are not limited to, members of the fibroblast growth factor family, including acidic and basic fibroblast growth factor (FGF-1 and -2) and FGF-4, members of the platelet-derived growth factor (PDGF) family, including PDGF-AB, PDGF-BB and PDGF-AA; EGFs, members of the insulin-like growth factor (IGF) family, including IGF-I and -II;, the TGF-β superfamily, including TGF-β1, 2 and 3 (including MP-52), osteoid-inducing factor (OIF), angiogenin(s), endothelins, hepatocyte growth factor and keratinocyte growth factor; members of the bone morphogenetic proteins (BMPs) BMP-1; BMP-3; BMP-2; OP-1; BMP-2A, -2B, -4, -7 and -14; HBGF-1 and HBGF-2; growth differentiation factors (GDFs), members of the hedgehog family of proteins, including indian, sonic and desert hedgehog; ADMP-1; GDF-5; and members of the colony-stimulating factor (CSF) family, including CSF-1, G-CSF, and GM-CSF; and isoforms thereof.

In some embodiments, the growth factor is selected from the group consisting of TGF-β, bFGF, and IGF-1. These growth factors are believed to promote regeneration of the nucleus pulposus, or stimulate proliferation and/or differentiation of chondrocytes, as well as ECM secretion. In one embodiment, the growth factor is TGF-β. More preferably, TGF-β is administered in an amount of between about 10 ng/ml and about 5000 ng/ml, for example, between about 50 ng/ml and about 500 ng/ml, *e.g.*, between about 100 ng/ml and about 300 ng/ml.

In some embodiments, platelet concentrate is provided as an additional therapeutic agent. In one embodiment, the growth factors released by the platelets are present in an amount at least two-fold (*e.g.*, four-fold) greater than the amount found in the blood from which the platelets were taken. In some embodiments, the platelet concentrate is autologous. In some embodiments, the platelet concentrate is platelet rich plasma (PRP). PRP is advantageous because it contains growth factors that can restimulate the growth of the ECM, and because its fibrin matrix provides a suitable scaffold for new tissue growth.

In one embodiment, the additional therapeutic agent is an antagonist of NO synthase. In one embodiment, the antagonist is L-NIL and N^{G} - monomethyl-L-arginine. In one embodiment, the additional therapeutic agent is an antagonist of PLA₂. In one embodiment, the additional therapeutic agent is a high specificity anti-proliferative agent, such as a high specificity anti-proliferative agent comprising rapamycin, a cdk inhibitor, a statin, and an anti-oxidant. In yet another embodiment, the anti-oxidant comprises a super oxide dismutase.

In one embodiment, the additional therapeutic agent is a highly specific apoptosis inhibitor selected from the group consisting of an EPO mimetic peptide and an EPO mimetic antibody fusion protein. In one embodiment, the additional therapeutic agent is a highly specific apoptosis inhibitor selected from the group consisting of: IGF-I and IGF-II. In one embodiment, the additional therapeutic agent is a highly specific caspase inhibitor. In one embodiment, the additional therapeutic agent comprises glycosaminoglycans.

In one embodiment, the formulation further comprises a liposomal delivery system.

In some embodiments, the formulation comprises a suitable biocompatible carrier such as saline. In some embodiments, the carrier is selected from the carriers disclosed in US Patent No. 6,277,969 ("Le"), the specification of which is incorporated by reference in its entirety.

In some embodiments, the formulation is administered through a drug pump.

Also in accordance with the present invention, there is provided a kit comprising a device containing an antagonist present in an amount effective to at least partially repair a degenerating disc.

Also in accordance with the present invention, there is provided a formulation for treating degenerative disc disease, comprising:
a) an antagonist, and
b) a growth factor

In some preferred embodiments, the antagonist is combined in the formulation with a viscosupplement. The viscosupplement has characteristics substantially similar to that of natural healthy nucleus pulposus ECM.

Preferably, the viscosupplement comprises glycosaminoglycans (GAGS). GAGS are biopolymers consisting of repeating polysaccharide units, and are present in nature on the cell surface as well as in the extracellular matrix of animals. GAGS are long unbranded polysaccharides containing a repeating disaccharide unit. The disaccharide unit contains either of two modified sugars, N-acetylgalactosaimne or N-acetylglucosamine and a uronic acid such as glucuronate or iduronate. GAGS are highly negatively charged molecules, with extended conformation that imparts high viscosity to the solution. In addition, to high viscosity, GAGS routinely possess low compressability, which makes these molecules ideal for a lubricating fluid in the joints. At the same time, their rigidity provides structural integrity to cells and provides passageways between cells, allowing for cell migration.

Recent work has shown that plasmid DNA will not elicit an inflammatory response as does the use of viral vectors. Genes encoding cartilage (anabolic) agents such as BMP may be efficacious if injected into the joint. In addition, overexpression of any of the growth factors provided herein or other agents such as a tissue inhibitor of MMPs (TIMP) that would limit local MMP activity would have positive effects on chondrocyte and ECM protection. In some embodiments, the TIMP is selected from the group consisting of TIMP-1 and TIMP-2. In some embodiments, the TIMP is autologous and is concentrated by filtration, centrifugation or by immuno-attachment processes. In other embodiments, the TIMP is manufactured recombinantly, and is preferably present in a concentration of at least 1000 times that found in the patient. In one embodiment, the plasmid contains the genetic code for human TGF-β or EPO.

Hyaluronic acid (HA) is a high molecular weight polysaccharide of N-acetyl glucosaime and glucuronic acid molecules that is naturally occurring in all mammals in a variety of tissue and some bacterial species. For the purposes of this invention, HA includes any derivatives such as hyaluronan and hyaluronic acid itself with H+ ion attached to the COO- group, and salts of hyaluronic acid whereby another positive ion replaces the H+ ion, as for example, with Na+ which forms sodium hyaluronate. Also included in the definition of HA is any physically or chemically cross-linked hyaluronic acid or derivative. HA is unique among the GAGS in that it does not contain any sulphate and is not found covalently attached to proteins as a proteoglycan. HA polymers are very large, with molecular weights of between about 100,000 and 10,000,000, and can displace a large volume of water. For the purposes of the present invention, a preferred embodiment includes a non-cross linked HA with a molecular weight of 0.5 - 10 M Dalton.

Preferably, the viscosupplement is selected from the group consisting of hyaluronic acid and hyaluronate (either cross-linked or uncross-linked). In some embodiments, the viscosupplement is ARTHREASE^{™} (DePuy Ltd., Leeds, U.K.). In some embodiments, the viscosupplement is a hyaluronic acid selected from the hyaluronic acids disclosed in U.S. Serial No. 09/298,539, entitled "Method of Treating Diseased, Injured or Abnormal Cartilage with Hyaluronic Acid and Growth Factors" (Radomsky *et al*.), the specification of which is incorporated by reference in its entirety.

Recent work has shown that plasmid DNA will not elicit an inflammatory response as does the use of viral vectors. Genes encoding cartilage (anabolic) agents such as BMP may be efficacious if injected into the joint. In addition, overexpression of any of the growth factors provided herein or other agents such as TIMP which would limit local MMP activity would have positive effects on chondrocyte and ECM protection. In one embodiment, the plasmid contains the genetic code for human TGF-β or EPO.

Because the causes of low back pain may be myriad, and because of the significant cost of some antagonists, it would be useful for a clinician to first perform a diagnostic test in order to confirm that the targeted disc in fact possesses high levels of the targeted proteins (e.g., proinflammatory cytokines and/or MMPs) prior to providing the injection.

In one embodiment, the diagnostic test comprises a non-invasive diagnostic test comprising, for example, using an MRI. In some embodiments, the MRI is able to quantify the aggrecans levels within the disc.

In one embodiment, the clinician would first perform a discogram in order to identify which disc or discs are responsible for the patient's low back pain. Next, the clinician would perform an invasive or non-invasive test upon the targeted disc in order to confirm the presence of or quantify the level of the proinflammatory cytokines and/or MMPs.

In one embodiment, the diagnostic test comprises an invasive test in which a portion of the disc is removed and analysed. In some embodiments, the clinician removes a portion of the nucleus pulposus. In some embodiments, the clinician removes a portion of the annulus fibrosus. Preferably, the removed material is a portion of the nucleus pulposus. The presence of proinflammatory cytokines and/or MMPs in the removed material may detected by procedures including, but not limited, to electrophoresis, or an enzyme-linked immunoabsorbent assay (ELISA) (as per Burke, Br. JBJS, 84-B(2), 2002).

In some embodiments, the diagnostic methods disclosed in U.S. Patent No. 6,277,969 ("Le") are selected. In these methods, high specificity anti- TNF-α compounds are used as diagnostic tools for detecting TNF-α in the patient known or suspected to have a high level of TNF-α.

After determining the levels of the different proinflammatory cytokines and/or MMPs in the degenerating disc, the clinician will preferably proceed to compare these diagnosed levels against pre-determined levels of the protein or proteins (such as cytokines or MMPs). If the diagnosed level of the proinflammatory cytokines and/or MMPs exceeds the pre-determined level, then the clinician may conclude that these higher levels are causing unwanted inflammatory action and proceed to directly inject a desired antagonist (e.g., a high specificity p38 kinase inhibitor) into the disc capable of inhibiting the targeted protein.

In some embodiments, a bioMEMS device containing a "lab on a chip" used in the diagnostic test.

In some embodiments, the predetermined level for an interleukin is at least 100 pg/ml. In some embodiments, the predetermined level for IL-6 is at least 250 pg/ml. In some embodiments, the predetermined level for IL-8 is at least 500 pg/ml. In some embodiments, the predetermined level for PGE2 is at least 1000 pg/ml. In some embodiments, the predetermined level for TNF-α is at least 500 pg/ml. In others, the predetermined level for TNF-α is at least 20 pg/ml, more preferably at least 30 pg/ml, more preferably at least 50 pg/ml, more preferably at least 1 ng/ml. In others, the predetermined level for TNF-α is at least 1 ng/disc, and in others, it is at least 1000 pg/disc.

It would also be useful to be able to determine whether directly administering the therapeutic substances of the present invention was, in fact, efficacious. Accordingly, one can measure the level of cytokines or MMPs remaining in the disc after administration.

### EXAMPLE I

This non-limiting prophetic example describes how to transdiscally administer a formulation comprising an HAAMMP and saline into a nucleus pulposus of a degenerating disc.

First, a clinician uses a diagnostic test to verify that a particular disc within a patient has high levels of MMPs.

Next, the clinician provides a local anesthetic (such as 5 ml lidocaine) to the region dorsal of the disc of concern to reduce subcutaneous pain.

Next, the clinician punctures the skin of the patient dorsal the disc of concern with a relatively large (e.g., 18-19 gauge) needle having a stylet therein, and advances the needle through subcutaneous fat and dorsal sacrolumbar ligament and muscles to the outer edge of the intervertebral disc.

Next, the stylet is removed from the needle.

Next, the clinician receives a syringe having a smaller gauge needle adapted to fit within the larger gauge needle. This needle is typically a 22 or 24 gauge needle. The barrel of the syringe contains the formulation of the present invention.

The formulation contains an HAAMMP, and has an HAAMMP concentration of between about 30 mg/ml and about 60 mg/ml.

Next, the physician advances the smaller needle co-axially through the larger needle and past the distal end of the larger needle, thereby puncturing the annulus fibrosus. The smaller needle is then further advanced into the center of the nucleus pulposus. Finally, the clinician depresses the plunger of the syringe, thereby injecting between about 0.1 and 1 ml of the formulation into the nucleus pulposus.

### EXAMPLE II

This non-limiting prophetic example is substantially similar to that of Example I, except that the formulation comprises a sustained release device comprising the co-polymer poly-DL-lactide-co-glycolide (PLG). The formulation contains HAAMMP as the antagonist, and has an HAAMMP concentration of between about 30 mg/ml and about 60 mg/ml.

### EXAMPLE III

This non-limiting prophetic example describes how to transdiscally administer a formulation comprising a HSCA and saline into a nucleus pulposus of a degenerating disc.

First, a clinician uses a diagnostic test to verify that a particular disc within a patient has high levels of a particular pro-inflammatory cytokine.

Next, the clinician provides a local anesthetic (such as 5 ml lidocaine) to the region dorsal of the disc of concern to reduce subcutaneous pain.

Next, the clinician punctures the skin of the patient dorsal the disc of concern with a relatively large (e.g., 18-19 gauge) needle having a stylet therein, and advances the needle through subcutaneous fat and dorsal sacrolumbar ligament and muscles to the outer edge of the intervertebral disc.

Next, the stylet is removed from the needle.

Next, the clinician receives a syringe having a smaller gauge needle adapted to fit within the larger gauge needle. This needle is typically a 22 or 24 gauge needle. The barrel of the syringe contains the formulation of the present invention.

The formulation contains REMICADE^{®} infliximab, and has an infliximab concentration of between about 30 mg/ml and about 60 mg/ml.

Next, the physician advances the smaller needle co-axially through the larger needle and past the distal end of the larger needle, thereby puncturing the annulus fibrosus. The smaller needle is then further advanced into the center of the nucleus pulposus. Finally, the clinician depresses the plunger of the syringe, thereby injecting between about 0.1 and 1 ml of the formulation into the nucleus pulposus.

### EXAMPLE IV

This non-limiting prophetic example is substantially similar to that of Example III, except that the formulation comprises a sustained release device comprising the co-polymer poly-DL-lactide-co-glycolide (PLG). The formulation contains infliximab as the antagonist, and has an infliximab concentration of between about 30 mg/ml and about 60 mg/ml.

While this invention has been particularly shown and described with references to preferred embodiments thereof, it will be understood by those skilled in the art that various changes in form and details may be made therein without departing from the scope of the invention encompassed by the appended Claims.

### EXAMPLE V

This non-limiting prophetic example describes how to administer transdiscally a formulation comprising a cycline compound into a nucleus pulposus of a degenerating disc.

First, a clinician uses a diagnostic test to verify that a particular disc within a patient has high levels of a particular pro-inflammatory cytokine and/or MMPs.

Next, the clinician provides a local anesthetic (such as 5 ml lidocaine) to the region dorsal of the disc of concern to reduce subcutaneous pain.

Next, the clinician punctures the skin of the patient dorsal to the disc of concern with a relatively large (e.g., 18-19 gauge) needle having a stylet therein, and advances the needle through subcutaneous fat and dorsal sacrolumbar ligament and muscles to the outer edge of the intervertebral disc.

Next, the stylet is removed from the needle.

In one embodiment, the portion of the nucleus pulposus is removed prior to administering the cycline compound.

Next, the clinician receives a syringe having a smaller gauge needle adapted to fit within the larger gauge needle. This needle is typically a 22 or 24 gauge needle. The barrel of the syringe contains the formulation of the present invention.

The formulation contains about 50 mg/ml of doxycycline.

Next, the physician advances the smaller needle co-axially through the larger needle and past the distal end of the larger needle, thereby puncturing the annulus fibrosus. The smaller needle is then further advanced into the center of the nucleus pulposus. Finally, the clinician depresses the plunger of the syringe, thereby injecting between about 0.1 and about.1 ml of the formulation into the nucleus pulposus.

### EXAMPLE VI

This non-limiting prophetic example is substantially similar to that of Example I, except that the formulation comprises a sustained release device comprising the co-polymer poly-DL-lactide-co-glycolide (PLG). The formulation contains doxycycline.

While this invention has been particularly shown and described with references to preferred embodiments thereof, it will be understood by those skilled in the art that various changes in form and details may be made therein without departing from the scope of the invention encompassed by the appended Claims.

## Claims

1. A formulation for use in a method of treating degenerative disc disease in an intervertebral disc having a nucleus pulposus, in which the formulation comprises a high specificity p38 kinase inhibitor administered in an effective amount to inhibit degradation of an extracellular matrix of the nucleus pulposus, the method involving administering the formulation transdiscally into the intervertebral disc.

2. A formulation as claimed in claim 1, in which the p38 kinase inhibitor is selected from the group consisting of:
i) diaryl imadazole;
ii) N,N'-diaryl urea;
iii) N,N-diaryl urea;
iv) benzophenone;
v) pyrazole ketone;
vi) indole amide;
vii) diamides;
viii) quinazoline;
ix) pyrimido [4,5-d]pyrimidinone; and
x) pyridylamino-quinazolines.

3. A formulation as claimed in claim 1, in which the p38 kinase inhibitor is an 1-aryl-2-pyridinyl heterocycle.

4. A formulation as claimed in claim 1, which is administered in the degenerative disc disease treatment method in an amount effective to inhibit degradation of the extracellular matrix of the nucleus pulposus.

5. A formulation as claimed in claim 1, which is administered in a dosage to produce a local tissue concentration of between about 5 µg/kg and about 50 ug/kg.

6. A formulation as claimed in claim 1, which is administered in the degenerative disc disease treatment method in an amount of less than about 1cc.

7. A formulation as claimed in claim 1, in which the p38 kinase inhibitor is present in an amount of no more than about 0.5 mg.

8. A formulation as claimed in claim 1, in which the p38 kinase inhibitor is present in the formulation in an amount of at least about 100 mg/ml.

9. A formulation as claimed in claim 1, which is administered in the degenerative disc disease treatment method through a drug pump.

10. A formulation as claimed in claim 1, which is administered in the degenerative disc disease treatment method through a needle.

11. A formulation as claimed in claim 1, which is administered in the degenerative disc disease treatment method in a volume of between about 0.03 ml and about 0.3 ml.

12. A formulation as claimed in claim 1, which includes a sustained release device.

13. A formulation as claimed in claim 12, in which the sustained release device has load-bearing capabilities.

14. A formulation as claimed in claim 13, in which the sustained release device comprise a hydrogel.

15. A formulation for use in a method of treating degeneration of an extracellular matrix of a nucleus pulposus in an intervertebral disc by injection of the formulation into the nucleus pulposus, in which the formulation comprises a p38 kinase inhibitor, wherein the injection of the formulation into the nucleus pulposus of the intervertebral disc inhibits degradation of the extracellular matrix of the nucleus pulposus.
